# EUROPEAN PATENT APPLICATION

(11) **EP 1 598 364 A1**
(43) Date of publication of application: **23.11.2005**
(21) Application number: 04012117.0
(22) Date of filing: 21.05.2004
(51) Int. Cl.: C07K 14/54, C12N 15/62, A61K 38/20

(54) **Chimerical soluble hyper IL-11 receptor and use thereof**

(71) Applicant: AGIRx Limited, Calvington, East Sussex BN 27 3TD (GB)
(72) Inventor: Mackiewicz, Andrzej, Prof., 61051 Poznan (PL); Dams-Kozlowska, Hanna, Dr., 61414 Poznan (PL); John, Stephan, Rose, Prof. Dr., 24211 Schellhorn (DE)
(74) Representative: Zwicker, Jörk

(57) **Abstract**

The present invention concerns a new designer cytokine termed H11, which is constructed by fusion of two soluble components, soluble interleukin 11 receptor (sIL-11 R) and interleukin 11 (IL-11) using their natural sequence and its use for the production of a medicament for treating or preventing a disease selected from the group consisting of a proliferative disease, a cytopathy, radiation damage, an IL-11 dependent inflammatory disorder, IL-11 dependent degenerative disorder and IL-11 dependent or mediated soft tissue disorder.

## Description

### Field of the invention

The present invention concerns a new designer cytokine termed H11, which is constructed by fusion of two soluble components, soluble interleukin 11 receptor (sIL-11 R) and interleukin 11 (IL-11) using their natural sequence and its use for the production of a medicament for treating or preventing a disease selected from the group consisting of a proliferative disease, a cytopathy, radiation damage, an IL-11 dependent inflammatory disorder, IL-11 dependent degenerative disorder and IL-11 dependent or mediated soft tissue disorder.

### Background of the invention

IL-11 is a functionally pleiotropic cytokine (reviewed in Du *et al.,* 1997). Originally it was identified in 1990 as a molecule promoting growth of the IL-6-dependent mouse plasmacytoma cell line (Paul *et al.,* 1990). It has been demonstrated later, that IL-11 exhibits multiple effects not only on the hematopoietic system, but that it also acts on many different cell types of the liver, gastrointestinal tract, lung, heart, central nervous system, bone, joint and immune system (reviewed in Du *et al.,* 1997 and Schwertschlag *et al.,* 1999). IL-11 acts synergistically with other growth factors in stimulating various stages of hematopoiesis both *in vitro* and *in vivo* including effects on progenitor cells, megakaryocytopoiesis and thrombocytopoiesis, erythropoiesis, myelopoiesis. It also affects the hematopoietic microenvironment (Du *et al.,* 1997). Due to its hematopoietic activity, IL-11 has been tested in severe chemotherapy-induced and bone-marrow transplantation-induced thrombocytopenia. Experiments in animal models have revealed that IL-11 stimulates thrombocytopoiesis (Hawley *et al.,* 1996) and accelerates recovery of peripheral blood neutrophils and platelets after bone marrow transplantation (Du *et al.,* 1993). Phase I and phase II clinical trials in cancer patients have demonstrated the potential of IL-11 in accelerating multilineage hematopoietic recovery and reducing thrombocytopenia (Gordon *et al.,* 1996, Tepler *et al.,* 1996, Isaacs *et al.,* 1997). IL-11 has already been approved by the FDA in the U.S.A. for treatment of chemotherapy induced thrombocytopenia. It has been demonstrated, that IL-11 displays anti-inflammatory activities due its ability to inhibit nuclear translocation of nuclear factor-_{κ}B (NF-_{κ}B) (Trepicchio et. Al, 1997), which decreases the production of proinflammatory cytokines secreted by macrophages such as tumor necrosis factor (TNF), IL-1β, IL-6, IL-12 (Trepicchio *et al.,* 1996, Leng *et al.,* 1997, Redlich *et al.,* 1996). Numerous studies in animal models and clinical trials have demonstrated the efficacy of IL-11 in treating inflammatory disorders including inflammatory bowel disease (Peterson *et al.,* 1998), radiation-induced lung damage (Redlich et al, 1996), sepsis (Chang *et al.,* 1996), rheumatoid arthritis (Anguita *et al.,* 1999, Walmsley *et al.*, 1998), inflammatory liver disease (Bozza *et al.,* 1999), mucositis (Sonis *et al.,* 1997) and psoriasis (Trepicchio *et al.,* 1999). Thus, IL-11 is considered as potential therapeutic agent in various inflammatory disorders. As mentioned above, IL-11 also demonstrates multiple biological activities outside the lymphohematopoietic system. These include induction of the acute phase protein synthesis in hepatocytes (Baumman *et al.,* 1991), suppression of lipoprotein lipase activity in adipocytes (Ohsumi *et al.,* 1994), induction of the differentiation in immortalized hippocampal neurons (Mehler *et al.,* 1993), participation in the development of osteoclastic cells (Girasole *et al.,* 1994), stimulation of the production of tissue inhibitor of metalloproteinases (Maier *et al.,* 1993). Moreover, it has been demonstrated that IL-11 plays an important role in female fertility, since mice lacking IL-11 R are infertile due to a defective uterine response to implantation (Robb *et al.,* 1998). The expression of IL-11 and IL-11 R in human uterine endometrium during the menstrual cycle indicates that IL-11 activity may be a key factor in human female fertility (Dimitriadis *et al.,* 2000, Karpovich *et al.,* 2003).

IL-11 together with IL-6, Leukaemia Inhibitor Factor (LIF), Oncostatin M (OSM), Ciliary Neutrophic Factor (CNTF), Cardiotrophin 1 (CT-1) belongs to the family of hemopoietic cytokines (named IL-6-type or gp130 cytokines), which share structural similarity and a common receptor subunit (gp130) (Bazan *et al.,* 1990). Although, each of the IL-6-type cytokines requires specific (unique) receptor complex, at least one molecule of gp130 is always engaged. Initially a ligand (IL-6, IL-11, CNTF) binds specifically to its α non-signaling receptor subunit and next recruits the signaling receptor chain. IL-6 and IL-11 use a gp130 homodimer for transducing the signal, while LIF, CNTF, CT-1 utilize a heterodimer gp130/LIFR. OSM either recruits gp130/OSMR or gp130/LIFR heterodimers (reviewed in Heinrich *et al.,* 2003, Bravo *et al.,* 2000).

The tertiary structure of IL-6-type cytokines has been intensely investigated during recent years. Crystal structures have been determinated for LIF (Robinson *et al.,* 1994), CNTF (McDonald *et al.,* 1995), IL-6 (Somers *et al.,* 1997) and OSM (Deller *et al.,* 2000). These studies revealed that each ligand exhibits the long chain four-helix bundle topology, which comprises four tightly packed α-helices (named A, B, C and D) ranging from 15 to 22 amino acids in length. The helices are connected in an up-up-down-down arrangement by the polypeptide loops. The A-B and C-D loops are relatively long as they connect parallel helices, whereas the B-C loop is shorter as it connects a pair of antiparallel helices. Detailed structural analysis and mutagenesis studies of IL-6-type cytokines have identified three receptor binding sites (termed I, II, III), which seem to be conserved among the gp130 family (reviewed in Bravo *et al.,* 2000). Site I, which enables ligand to bind to its non-signaling receptor, is formed by amino acids from the C-terminal part of the A-B loop and the C-terminal residues of the D helix. Site II seems to be a universal gp130 binding site for all members of IL-6-type cytokines and consists of exposed residues on helices A and C. Site III is composed of an N-terminal half of helix D, the N-terminal part of the A-B loop and amino acid residues of the end of the C-D loop. This site is always occupied by the second signaling receptor: gp 130, LIFR or OSMR, depending upon the identity of the ligand.

The receptors involved in IL-6-type cytokine signaling belong to the type I membrane proteins. They posses an extracellular N-terminus and one transmembrane domain (with the exception of CNTFR, which is linked to the membrane by a lipid anchor (Davis *et al.,* 1991). Because of a common structural motif in their extracellular region, they are classified as cytokine receptor class I family (Bazan *et al.,* 1990). This family is characterized by the presence of at least one cytokine binding homology domain (CHD) consisting of two fibronectin-type-III-like domains (FNIII) termed D2 and D3. The CHD is composed of approximately 200 amino acids, with four positionally conserved cysteine residues at the N-terminal domain and a characteristic conserved Trp-Ser-X-Trp-Ser (WSXWS) motif at the C-terminal domain. Additionally each receptor subunit contains an Ig-like domain, which is located at the N-terminus of the membrane-proximal CHD. The IL-6-type receptors are divided into two groups: α and β subunits. Receptors α (for IL-6, IL-11 and CNTF) are not involved in signal transduction. Subunits β, the signal transducing receptor chains, contain a considerably larger cytoplasmic part than α subunits and have three membrane-proximal FNIII domains that may play some role such as in stabilization and/or in orientation of the transmembrane receptor dimers (reviewed in Bravo *et al.,* 2000, Heinrich *et al.,* 2003). Besides the membrane bound IL-6-type receptor subunits, their soluble forms were found in biological fluids (reviewed in Marz *et al.,* 1999). They are formed either by limited proteolysis (shedding) of membrane-bound receptors or by translation from differential spliced mRNA.

According to the structural model proposed by Czupryn *et al.* IL-11 has a four-helix bundle topology (Czupryn *et al.,* 1995). Extensive structural analysis and mutagenesis studies have determinated three receptor binding sites, analogous in location to sites I, II, and III of IL-6 or CNTF. These sites have been characterized in murine as well as in human IL-11 (Barton *et al.,* 1999, Tacken *et al.,* 1999, respectively). Site I enables IL-11 to bind to αIL-11 R, whereas sites II and III mediate binding to two different gp130 molecules. Carboxyl-terminal deletion mutagenesis studies have demonstrated that removal of the last 4 amino acids reduces the activity of recombinant human IL-11 (rhIL-11) 25-fold while elimination of 8 or more C-terminal residues completely abolishes its activity (Czupryn *et al.,* 1995). Detailed studies on ligand-receptor interaction sites enabled a creation of a new potent IL-11 antagonist and agonist (Underhill-Day *et al.,* 2003, Harmegnies *et al.,* 2003, respectively). Above molecules were created by replacing one or two amino acids with other amino acids, which led to the inhibition and increase, respectively, of the ligand/receptor formation.

IL-11 R α subunits have been described in mouse and human (Hilton *et al.,* 1994, Cherel *et al.,* 1995 and Nandrukar *et al.,* 1996, respectively). Two isoforms of the human IL-11 R α-chain have been identified (Cherel *et al.,* 1996). They differ in their cytoplasmic domain, whereas extracellular and transmembrane parts are identical. The first isoform (IL-11 R α1) has a short cytoplasmic domain and the other (IL-11 α2) lacks this region. Both isoforms demonstrated similar functions and properties when tested on Ba/F3 cells transfected with gp130 (Lebeau *et al.,* 1997). Structurally the extracellular part of the a IL-11 R can be divided into three domains: Ig-like domain (D1), and one CHD consisting of two FNIII domains (D2 and D3) (Cherel *et al.,* 1995). There is little known about amino acid residues in IL-11 R α-chain involved in ligand binding, since no structural information is available for the CHD of IL-11 R so far. A recent study of Schleinkofer *et al.* demonstrated that the D3 domain is mainly involved in ligand interaction, since binding capacity of this domain is as high as that of full length receptor (Schleinkofer et al, 2001). However, their molecular model of IL-11/IL-11 R complex, which has been built on the basis of X-ray structures of human growth hormone and its receptor complex (de Vos *et al.,* 1992) and human CNTF structure (McDonald *et al.,* 1995) as a template, demonstrated that regions of IL-11 R that bind with site I of IL-11 are located within domains D2 and D3. Based on this model, as well as on analogy with IL-6/IL-6 R interaction, where the D3 domain is sufficient for ligand binding but not for gp130 association (Ozbek *et al.,* 1998), Schleinkefer group postulated that D2 domain seems to be involved in: (i) interdomain stabilization, (ii) supplying specificity or (iii) formation of an interface to the gp130 subunit or (iv) it is needed to induce a conformational change in the site III of the ligand required for interaction with gp130 (Schleinkofer et al, 2001). The role of Ig-like domain of IL-11 R is not defined, while in the case of IL-6 R although it is not required for assembly of functional receptor (Yawata *et al.,* 1993), it stabilizes the receptor during intracellular trafficking (Vollmer *et al.,* 1999).

The stoichiometry of the high affinity IL-11 receptor complex has been determined *in vitro* as a hexameric complex consisting of two IL-11 molecules, two IL-11 Rα chains and homodimer of gp130 molecules (Barton *et al.,* 2000). It was predicted that IL-11 binds αIL-11 R through site I, CHD of gp130 through site II and the Ig-like domain of the second gp130 through site III. However, the stoichiometry of the IL-11 ligand/receptor complex is still under dispute. Grotzinger *et al.* have suggested that a tetrameric complex is formed, in which IL-11 first binds to the CHD of the specific α receptor and that a tetramer assembles next via sites II and III of the cytokine with two molecules of gp130 (Grotzinger *et al.,* 1997). In the IL-6 case, the tetramer is able to bind additional IL-6/IL-6 R complex, forming a hexameric, but non signaling complex (Grotzinger *et al.,* 1999). Neddermann *et al.* proposed formation of a pentameric complex *in vitro* composed of two IL-11, two IL-11 R and one gp130 molecule. However, they suggested that there is another unidentified β chain, which contributes to the formation of the hexameric receptor complex (Neddermann *et al.,* 1996). Harmegnies *et al.* supported above data recently (Harmegnies *et al.,* 2003). They created a IL-11 mutein with an increased hydrophobicity at site I, which proved to be a potent IL-11 agonist and revealed a diverse biological activity. Depending on the cellular model used in the assessment of the activity the mutated IL-11 demonstrated increased or reduced agonistic properties when compared to the wild type IL-11. On the other hand, studies on IL-11 antagonists supported the hexameric complex proposed by Barton *et al.* (Underhill-Day *et al.,* 2003).

The soluble form of IL-11 R α chain (sIL-11 R) has been postulated to exist, although it has not been found in body fluids so far. Its existence is based on the identification of a transcript potentially encoding a soluble form of IL-11 R (Robb *et al.,* 1996). The recombinant sIL-11 R acts *in vitro* as IL-11 agonist (Baumann *et al.,* 1996, Neddermann *et al.,* 1996, Karow *et al.,* 1996, Curtis *et al.,* 1997). In addition, sIL-11 R not only potentiated effects of IL-11 on cells that are normally responsive to IL-11, but also mediated signal transduction in cells expressing gp130 molecules only in the presence of IL-11 (Baumann *et al.,* 1996, Karow *et al.,* 1996). However, the concentration of IL-11 required to mediate a biological response using sIL-11 R had to be 10 to 20-fold higher than using membrane receptor (Curtis *et al.,* 1997). Since the expression of IL-11 R is limited to certain cell types, while gp130 is present on all cells of the body, use of the sIL-11 R significantly widens the range of IL-11 bioactivity. Furthermore, it was observed that sIL-11 R can act as an IL-11 antagonist when tested on cells expressing the membrane bound form of IL-11 R and gp130 (Curtis *et al.,* 1997). The observed antagonism was due to the competition between recombinant sIL-11 R and transmembrane IL-11 R for IL-11 and/or was dependent on the number of gp130 molecules. These findings however, suggest that the physiological role of sIL-11 R is much more complex than sIL-6 R and that results obtained for sIL-6 R can not straight forwardly be applied to sIL-11 R.

In order to increase and modify potential bioactivity of some molecular agents, the idea of linkage of two soluble naturally existing components has been postulated. The fusion proteins are expected to be more stable and are needed in lower effective dose in supporting bioactivity. Such recombinant fusion proteins have been already described. The separately encoded subunits of IL-12 (p35 and p40) have been connected by a polypeptide linker (Lieschke *et al.,* 1997). Hyper-IL-6 is another example of a new designer agent, which consists of D2 and D3 domain of IL-6 R α chain connected to IL-6 via polypeptide linker (Fischer *et al.,* 1997, WO 97/32891). In the case of IL-6, it was observed that the effective concentration of IL-6 and sIL-6 R, which is needed for the stimulation of cells which lack membrane IL-6 R is very high (Rose-John *et al.,* 1990). Furthermore, the average half live of the IL-6/sIL-6 R complex might be shorter than the time needed to assemble the IL-6/sIL-6 R/gp130 complex (Wells *et al.,* 1996). The stability of IL-6/sIL-6 R complex was enhanced by linking both components in order to create a fusion protein (Hyper-IL-6) (WO 97/32891). Hyper-IL-6 can directly bind to its signal transducing receptor subunit and enhance IL-6 activity. Hyper-IL-6 is a fully active fusion protein, which mediates response at 10 to 1000-fold lower dose compared to the combination of soluble IL-6 and sIL-6 R molecules (Fischer *et al.,* 1997). In analogy, another superagonist has been designed for IL-6-type family named IL-11/R-FP (Pflanz *et al.,* 1999). IL-11/R-FP was created by covalently linking D2 and D3 domains of IL-11 R (position L/109 - G/318) with IL-11 (position P/29 - L/199) using a 21 amino acid linker and demonstrated 50 fold higher activity *in vitro* than the combination of IL-11 and sIL-11 R. However, this construct was composed of truncated segments of the human IL-11 R and IL-11 and, thus, lacks naturally existing parts of the respective receptor and cytokine. Moreover, the artificial linker used is a not naturally occurring sequence, which contributes to the immunogenicity of IL-11/R-FP when used for treatment of human patients.

Thus, one object of the present invention is the construction of a new designer cytokine Hyper IL-11 (H11), which is composed of naturally existing components, which elicits only little or no immunogenicity in a human host and which can provide an anti-tumor response *in vivo* which is superior to the prior art IL-11/R-FP.

A second object of this invention is the use of H11 for the treatment and/or prevention of proliferative diseases, cytopathies, radiation damage, IL-11 dependent inflammatory disorders, IL-11 dependent degenerative disorders, IL-11 dependent or mediated soft tissue disorders.

A third object of this invention is the construction of vector systems, in particular retroviral, lentiviral and adenoviral vectors caring H11 for modification of cancer cells as well as other mammalian cells and the use of such modified cells as, for example, gene modified tumor vaccine (GMTV) for treatment and/or prevention of cancers, in particular melanoma, renal cancer or pancreatic cancer.

Further objects of the present invention include the production of fusion protein H11 in various expression systems including Baculovirus expression systems and the use of this protein for the treatment or prevention of a proliferative disease, a cytopathy, radiation damage, an IL-11 dependent inflammatory disorder, IL-11 dependent degenerative disorder and IL-11 dependent or mediated soft tissue disorder.

### Summary of the Invention

Interleukin 11 (IL-11) is a pleiotropic cytokine initially identified as a factor stimulating proliferation of a IL-6-dependet murine plasmacytoma cell line T1165 (Paul *et al.,* 1990). Today IL-11 is known to interact with a variety of hematopoietic and non-hematopoietic cell types (Du *et al.,* 1997).

IL-11 receptor complex comprises a specific α subunit and gp130. It was demonstrated that sIL-11 R is agonistic to IL-11 similarly as sIL-6 R to IL-6 (Baumann *et al.,* 1996). Moreover, it was shown that transduction of IL-11 into B78H1 cells, a murine melanoma cell line, inhibited tumor growth (Dams-Kozlowska *et al.,* 2000). However, a mixture of sIL-11 R modified B78H1 cells and IL-11 secreting B78H1 cells had no superior effect on tumor growth, if compared to IL-11 alone. In order to enhance IL-11 activity *in vivo* we designed a new cytokine termed Hyper IL-11 (H11), which showed an improved *in vivo* anti-tumor activity. Construction of H11 is based on linking soluble form of αIL-11 R (extracellular part of αIL-11 R including its signal sequence in order to provide secreting) with IL-11 sequence (without its signal sequence).

Thus the invention provides in one aspect a polynucleotide selected from the group consisting of:
(a) polynucleotides encoding a fusion interleukin-11 receptor (IL-11 R) and IL-11 polypeptide (H11) comprising at least sIL-11 R having the deduced amino acid sequence as shown in SEQ ID NO: 1 and a mature IL-11 having the deduced amino acid sequence as shown in SEQ ID NO: 2;
(b) polynucleotides comprising the coding sequence of sIL-11 R, as shown in SEQ ID NO: 4 and the coding sequence of IL-11, as shown in SEQ ID NO: 5 encoding H11;
(c) polynucleotides encoding a fragment and/or derivative of a H11 encoded by a polynucleotide of any one of (a) to (b), wherein in said derivative one or more amino acid residues are conservatively substituted compared to said H11 and said fragment and/or derivative has *in vivo* anti-tumor activity;
(d) polynucleotides which are at least 50% identical to a polynucleotide as defined in any one of (a) to (c) and which code for a H11 having *in vivo* anti-tumor activity; and
(e) polynucleotides the complementary strand of which hybridizes, preferably under stringent conditions to a polynucleotide as defined in any one of (a) to (d) and which code for a H11 having *in vivo* anti-tumor activity;
or the complementary strand of such a polynucleotide.

In a preferred embodiment the polynucleotide encoding sIL-11 R is positioned 5' terminal with respect to the polynucleotide encoding mature IL-11.

In a preferred embodiment the nucleotide sequence intervening the polynucleotide sequences encoding the sIL-11 R and the mature IL-11 encodes a non-immunogenic peptide.

In a preferred embodiment the polynucleotides encoding sIL-11 R and mature IL-11 are directly linked.

In a preferred embodiment the polynucleotide is selected from the group consisting of:
(a) polynucleotides encoding H11 having the deduced amino acid sequence as shown in SEQ ID NO: 3;
(b) polynucleotides comprising the coding sequence of H11, as shown in SEQ ID NO: 6;
(c) polynucleotides encoding a fragment and/or derivative of a H11 encoded by a polynucleotide of any one of (a) to (b), wherein in said derivative one or more amino acid residues are conservatively substituted compared to said H11 and said fragment and/or derivative has *in vivo* anti-tumor activity;
(d) polynucleotides which are at least 50% identical to a polynucleotide as defined in any one of (a) to (c) and which code for a H11 having *in vivo* anti-tumor activity; and
(e) polynucleotides the complementary strand of which hybridizes, preferably under stringent conditions to a polynucleotide as defined in any one of (a) to (d) and which code for a H11 having *in vivo* anti-tumor activity;
or the complementary strand of such a polynucleotide.

In a preferred embodiment of the polynucleotide of the present invention the polynucleotide is DNA, genomic DNA or RNA.

A further aspect of the present invention is a vector containing at least one polynucleotide of the present invention.

In a preferred embodiment of the vector of the present invention the polynucleotide is operatively linked to expression control sequences allowing expression in prokaryotic and/or eukaryotic host cells.

In a preferred embodiment of the vector of the present invention the expression control sequences are selected from the group consisting of CMV, SV40, polyhedrin promoter, retroviral LTRs, phosphoglycerate kinase (PGK), elongation factor 1-α (EF1α) and phosphoenolpyruvate carboxykinase (PEPCK).

In a preferred embodiment of the vector of the present invention is selected from the group consisting of a plasmid; phagemid; phage; cosmid; artificial mammalian chromosome; artifical yeast chromosomes; knock-out or knock-in construct; viruses, in particular adenoviruses, vaccinia viruses, attenuated vaccinia viruses, canary pox viruses, lentiviruses, herpes viruses, in particular Herpes simplex virus, baculoviruses, retroviruses, adeno-associated-viruses (AAV), rhinoviruses, human immune deficiency viruses (HIV), filovirus and engineered versions thereof; virosomes; virus-like particles; and liposomes.

A further aspect of the invention is a host cell genetically engineered with the polynucleotide of the present invention or the vector of the present invention.

In a preferred embodiment the host cell is selected from the group consisting of insect cells, in particular *Trichoplusia ni* and *Spodoptera frugiperda;* mammalian cells, in particular stem cells, hepatocytes, adipocytes, neurons, osteoclasts, uterine endometrium cells, dermatocytes, myocardial cells, mucosal cells, hemopoietic cells or tumor cells like, for example the murine myeloma cell line - NSO; bacterial cells, in particular of *Escherichia* or *Bacillus* species and yeast cells, in particular of *Pischia* or *Saccharomyces* species.

In a more preferred embodiment the host cell is selected from the group consisting of renal cancer cells, melanoma cells, pancreatic cancer cells, leucocytes, packaging cells, in particular amphotropic or ecotropic packaging cells.

In a preferred embodiment of the host cell of the present invention the host cell is genetically engineered to express at least one further polynucleotide.

In a preferred embodiment of the host cell of the present invention the further polynucleotide encodes a cytokine, in particular GM-CSF, IL-6, IL-11, IL-15, EPO, interferons, LIF, OSM, CNTF, CT-1 and sIL-6 R/IL-6 fusion proteins (e.g. Hyper IL-6).

A further aspect of the present invention is a process for producing cells capable of expressing H11 comprising genetically engineering cells *in vitro* with the vector of the present invention, wherein said H11 is encoded by the polynucleotide of the present invention.

A further aspect of the present invention is a process for producing a H11 polypeptide encoded by the polynucleotide of the present invention comprising: culturing the host cell of the present invention and recovering the H11 polypeptide encoded by said polynucleotide.

A further aspect of the present invention is a H11 polypeptide having the amino acid sequence encoded by the polynucleotide of the present invention or obtainable by the process of the present invention.

A further aspect of the present invention is an antibody specific to the polypeptide encoded by the polynucleotide of the present invention or obtainable by the process of the present invention, which is essentially non-specific to sIL-11 R and IL-11.

A further aspect of the present invention is a pharmaceutical composition comprising the host cell of the present invention or obtainable according to the process the present invention or the H11 fusion polypeptide of the present invention or obtainable according to the process of the present invention, further comprising excipients, stabilizers, protectants, buffers and/or additives.

A further aspect of the present invention is the use of the host cell of the present invention or obtainable according to the process the present invention or the H11 fusion polypeptide of the present invention or obtainable according to the process of the present invention for the production of a medicament for the prevention or treatment of a disease selected from the group consisting of a proliferative disease, a cytopathy, radiation damage, an IL-11 dependent inflammatory disorder, IL-11 dependent degenerative disorders and IL-11 dependent or mediated soft tissue disorders.

In a preferred embodiment the proliferative disease is selected from the group consisting of cancer of the gastrointestinal or colorectal tract, liver, pancreas, kidney, bladder, prostate, endometrium, ovary, testes, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, glioma, astrocytoma, osteosarcoma, soft tissue sarcoma, hemangioma, endocrinological tumor, hematologic neoplasia including leukemia, lymphoma, and other myeloproliferative and lymphoproliferative diseases, carcinoma in situ, hyperplastic lesion, adenoma, fibroma, histiocytosis, chronic inflammatory proliferative disease, vascular proliferative disease and virus-induced proliferative disease; in particular melanoma, pancreatic and renal cancer.

In a preferred embodiment the cytopathy is selected form the group consisting of thrombocytopenia, hematocytopenia, and pancytopenia.

In a preferred embodiment the radiation damage is damage induced during radiation therapy, in particular during the treatment of proliferative diseases.

In a preferred embodiment the IL-11 dependent inflammatory disorder is selected from the group consisting of liver failure; hepatitis; hepatopathy; sepsis; chemotherapy or radiation-induced tissue damage, in particular lung damage; inflammatory diseases, in particular inflammatory bowel disease, rheumatoid arthritis, inflammatory liver disease; mucositis; allergies; endometriosis; vasculitis; vascular disease associated with endothelial inflammation, in particular ischaemic heart diseases or peripheral vascular disease; and psoriasis.

In a preferred embodiment the IL-11 dependent degenerative disorder is selected from the group consisting of degenerative CNS diseases, PNS diseases and osteoarthritis.

In a preferred embodiment the IL-11 dependent or mediated soft tissue disorder is selected from the group consisting of obesity and idiopathic female infertility.

In a further preferred embodiment a further cytokine is administered prior, simultaneously or subsequently to the administration of H11 or the H11 expressing host cell.

In a further aspect of the present invention is the use of H11 fusion polypeptide of the present invention or obtainable according to the process of the present invention for the production of a medicament for adjuvant therapy during stem cell therapy.

In a further aspect of the present invention is the use of the H11 fusion polypeptide of the present invention or obtainable according to the process of the present invention for the *in vitro* differentiation of cells, in particular stem or precursor cells.

### Detailed description of the invention.

A new designer cytokine Hyper IL-11 (H11) was constructed, which is composed of naturally existing components. It contains full length human sIL-11 R connected with mature human IL-11 using their natural sequences. Such construction has two major advantages: (i) its components are as close as possible to the natural wild type forms of both proteins and (ii) thus possible immunogenicity and other side effects due to a recombinant agent, which diverges from the naturally occurring molecules, i.e. is "less wild type", as known in the prior art are avoided.

Therefore in one aspect the invention provides a polynucleotide selected from the group consisting of:
(a) polynucleotides encoding a fusion interleukin-11 receptor (IL-11 R) and IL-11 polypeptide (H11) comprising at least sIL-11 R having the deduced amino acid sequence as shown in SEQ ID NO: 1 and a mature IL-11 having the deduced amino acid sequence as shown in SEQ ID NO: 2;
(b) polynucleotides comprising the coding sequence of sIL-11 R, as shown in SEQ ID NO: 4 and the coding sequence of IL-11, as shown in SEQ ID NO: 5 encoding H11;
(c) polynucleotides encoding a fragment and/or derivative of a H11 encoded by a polynucleotide of any one of (a) to (b), wherein in said derivative one or more amino acid residues are conservatively substituted compared to said H11 and said fragment and/or derivative has *in vivo* anti-tumor activity;
(d) polynucleotides which are at least 50% identical to a polynucleotide as defined in any one of (a) to (c) and which code for a H11 having *in vivo* anti-tumor activity; and
(e) polynucleotides the complementary strand of which hybridizes, preferably under stringent conditions to a polynucleotide as defined in any one of (a) to (d) and which code for a H11 having *in vivo* anti-tumor activity;
or the complementary strand of such a polynucleotide.

A fragment of H11 is a fusion protein carrying N-terminal and/or C-terminal deletions in one or both the IL11 or sIL-11 R part. However, deletions of the C-terminal part of IL-11 are not larger than 4 amino acids, preferably only 3, 2, or 1 amino acids. Deletions of the N-terminal part of IL-11 are less dramatic in their effect on the *in vivo* function of IL-11 in the context of H11 and, therefore, the N-terminal part of IL-11 within H11 can lack between 10 and 1 amino acids, albeit it is preferred, that the N-terminus lacks less than 10, less than 9, less than 8, less than 7, less than 6, less than 5, less than 4, less than 3, less than 2 amino acids.

Since the surprising advantage of the present H11 fusion polypeptide over prior art IL11 fusion polypeptides in part relies on the avoidance of creating new immunogenic epitopes the deletion of larger parts of sIL-11 R should be avoided, since this can both decrease the *in vivo* anti-tumor activity and increase the immunogenicity. Minimally the sIL-11 R molecule employed should comprise the D1, D2 and D3 domain of IL-11 R. The sIL-11 R protein can be deleted on its C-and/or N-terminus, preferably it can carry between 1 to 5 C-terminal, more preferably 4, 3, 2, or 1 amino acid deletions.

During maturation of both the sIL-11 R expressed alone and in the context of IL-11, i.e. as sIL-11 R-IL-11-fusion protein, about 22 amino acids are clipped of from the N-terminus of sIL-11 R. Consequently, the sIL-11 R preferably caries between 1 to 27 N-terminal amino acid deletions. It is further preferred that the N-terminus lacks less than 27, less than 26, less than 25, less than 24 and less than 23 amino acids.

A "derivative" of H11 is a polypeptide with no more than 20 (e.g., no more than: 20, 19, 18, 17, 16, 15, 14, 13, 12, 11, 10, nine, eight, seven, six, five, four, three, two, or one) conservative substitutions. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine. All that is required of a polypeptide having one or more conservative substitutions is that it has at least 10% (e.g., at least: 10%, 20%; 30%; 40%; 50%; 60%; 70%; 80%; 90%; 95%; 98%; 99%; 99.5%; or 100% or even more) of the ability of the designer cytokine H11 to elicit an anti-tumor response *in vivo.*

The determination of percent identity between two sequences is accomplished using the mathematical algorithm of Karlin and Altschul, 1993. Such an algorithm is incorporated into the BLASTN and BLASTP programs of Altschul *et al.,* 1990. To obtain gapped alignments for comparative purposes, Gapped BLAST is utilized as described in Altschul *et al.,* 1997. When utilizing BLAST and Gapped BLAST programs, the default parameters of the respective programs are used.

Hybridization can also be used as a measure of homology between two nucleic acid sequences. A nucleic acid sequence encoding a H11 polypeptide as disclosed herein, or a portion thereof, can be used as a hybridization probe according to standard hybridization techniques. Hybridization conditions are known to those skilled in the art and can be found in Current Protocols in Molecular Biology, John Wiley & Sons, N.Y., 6.3.1-6.3.6, 1991. Moderate hybridization conditions are defined as equivalent to hybridization in 2X sodium chloride/sodium citrate (SSC) at 30°C, followed by a wash in 1 X SSC, 0.1% SDS at 50°C. Highly stringent conditions are defined as equivalent to hybridization in 6X sodium chloride/sodium citrate (SSC) at 45°C, followed by a wash in 0.2 X SSC, 0.1% SDS at 65°C.

The *in vivo* anti-tumor activity of a H11 fusion protein of the present invention can be assessed by a variety of prior art methods including tumor growth in a mouse model grafted with tumor cells. One example of an assay system, which can be used to assess the *in vivo* anti-tumor response is disclosed in example 8 below. A H11 fusion polypeptide, which is considered to have *"in vivo* anti-tumor activity" should have at least 10%, preferably at least a 20%, 30%, 40%, 50%, 60%, 70%, 80%, 90%, 100% or more of the activity of the H11 polypeptide according to SEQ ID NO. 3.

Polynucleotides encoding H11 polypeptide as disclosed herein can be identified based on their similarity to the sequences set forth in SEQ ID No. 4 to 6. For example, the identification can be based on sequence identity. In certain preferred embodiments the invention features isolated nucleic acid molecules which are at least 50% (or 55%, 65%, 75%, 85%, 90%, 95%, or 98%) identical to: (a) a nucleic acid molecule that encodes the polypeptide of SEQ ID NOs: 1 to 3 or (b) the nucleotide sequence of SEQ ID NOs: 4-6; and code for a H11 polypeptide with *in vivo* anti-tumor activity.

In a preferred embodiment the polynucleotide encoding the sIL-11 R is positioned 5' terminal with respect to the polynucleotide encoding mature IL-11. This arrangement is preferred because it leaves the C-terminal region of IL-11 unobstructed by the fusion to sIL-11 R.

As pointed out above it is an object of the invention to provide a designer cytokine with a strong agonistic action, an *in vivo* anti-tumor effect and with low antigenicity. Therefore, in a preferred embodiment of the polynucleotide of the present invention, the nucleotide sequence between the polynucleotide sequences encoding the sIL-11 R and the mature IL-11 encodes an essentially non-immunogenic peptide. More preferably the polynucleotides which are connected by an essentially non-immunogenic peptide are arranged in such a manner that sIL-11 R is positioned 5'terminal with respect to the polynucleotide encoding mature IL-11. The term essentially "non-immunogenic peptide", refers to a short poly amino acid stretch of a length of 1 to 10 amino acids, preferably less than 9, less than 8, less than 7, less than 6, less than 5, less than 4, less than 3, less than 2 amino acids coupled through peptide bonds, which does not substantially increase the immune response against the sIL-11-R IL-11 fusion polypeptide, if compared to a similar fusion peptide with no intervening peptide.

The extent of an immune response can be assessed through a variety of art known methods including, for example, assays determining B-cell stimulation and the like. Another approach to assess the immunogenicity of the polypeptide linker is the immunization of a test animal with a H11 polypeptide of the present invention comprising a peptide linker between the sIL-11 R and the IL-11 part of the fusion polypeptide and the subsequent determination, if the test animal produces antibodies, which specifically recognize the peptide linker. The specificity of recognition is determined by comparison of the amount of binding observed to the peptide linker and the amount of binding observed for an unrelated protein. Examples of such unrelated proteins are proteins not endogenous to the test animal and include, e.g. bovine serum albumine, milk proteins and the like. If the polyclonal serum of the test animal exhibits a 5-fold or less, preferably 4-fold or less, more preferably 3-fold or less and most preferably 2-fold or less specificity to the peptide linker if compared to the unrelated protein, the peptide linker is considered essentially non-immunogenic. Examples of linkers which are essentially non-immunogenic are stretches of amino acids with small side chains like, for example, glycine, alanine, valine, threonine or serine and can be depicted by the following formula: Nₓ, wherein N independently at each position of the peptide chain with a length x has the meaning A, G, S, T or V and x has the meaning 1 to 10. Thus, in a preferred embodiment the linker is selected from the group consisting of: A, G, S, T, V; AA, AG, AS, AT, AV, GA, GG, GS, GT, GV, SA, SG, SS, ST, SV, TA, TG, TS, TT, TV, VA, VG, VS, VT, VV; AAA, AAG, AGA, GAA, AAS, ASA, SAA, AAT, ATA, TAA, AAV, AVA, VAA, GGG, GGA, GAG, AGG, GGS, GSG, SGG, GGT, GTG, TGG, GGV, GVG, VGG, SSS, SSA, SAS, ASS, SSG, SGS, GSS, SST, STS, TSS, SSV, SVS, VSS, VVV, VVA, VAV, AVV, VVG, VGV, GVV, VVS, VSV, SVV, VVT, VTV, TVV, AGS, ASG, GAS, GSA, SAG, SGA, AGT, ATG, GAT, GTA, TAG, TGA, AGV, AVG, GAV, GVA, VAG, VGA, AST, ATS, SAT, STA, TAS, TSA, ASV, AVS, SAV, SVA, VAS, VSA, GST, GTS, SGT, STG, TGS, TSG, GSV, GVS, SGV, SVG, VGS, VSG, STV, SVT, TSV, TVS,VST, and VTS.

In a particular preferred embodiment of the polynucleotide of the present invention the polynucleotides encoding sIL-11 R and mature IL-11 are directly linked. In this context "directly linked" means that no non-naturally occurring nucleotides encoding non-naturally occurring linker polypeptides are positioned between the sIL-11 R and the IL-11 encoding polynucleotide.

In a further preferred embodiment the polynucleotide is selected from the group consisting of:
(a) polynucleotides encoding H11 having the deduced amino acid sequence as shown in SEQ ID NO: 3;
(b) polynucleotides comprising the coding sequence of H11, as shown in SEQ ID NO: 6;
(c) polynucleotides encoding a fragment and/or derivative of a H11 encoded by a polynucleotide of any one of (a) to (b), wherein in said derivative one or more amino acid residues are conservatively substituted compared to said H11 and said fragment and/or derivative has *in vivo* anti-tumor activity;
(d) polynucleotides which are at least 50% identical to a polynucleotide as defined in any one of (a) to (c) and which code for a H11 having *in vivo* anti-tumor activity; and
(e) polynucleotides the complementary strand of which hybridizes, preferably under stringent conditions to a polynucleotide as defined in any one of (a) to (d) and which code for a H11 having *in vivo* anti-tumor activity;
or the complementary strand of such a polynucleotide.

The terms "fragment", "derivative" and "*in vivo* tumor activity" as used in the context of the preferred embodiment have the same meaning as outlined above.

H11 encoding nucleic acid molecules of the invention can be DNA, cDNA, genomic DNA, synthetic DNA or RNA, and can be double-stranded or single-stranded, the sense and/or an antisense strand. Segments of these molecules are also considered within the scope of the invention, and can be produced by, for example, the polymerase chain reaction (PCR) or generated by treatment with one or more restriction endonucleases. A ribonucleic acid (RNA) molecule can be produced, for example, by *in vitro* transcription.

The polynucleotide molecules of the invention can contain naturally occurring sequences, or sequences that differ from those that occur naturally, but, due to the degeneracy of the genetic code, encode the same polypeptide, i.e. the polypeptides with SEQ ID NOs: 1 to 3. The polynucleotide molecules can comprise additional polynucleotides at there 3' and/or 5' terminal end, which code for further polypeptides.

The polynucleotide of fusion polynucleotide molecules of the invention can be synthesized *in vitro* (for example, by phosphoramidite-based synthesis) or can be obtained from a cell, such as the cell of a bacteria, yeast, insect or mammal.

A further aspect of the present invention is a vector containing the polynucleotide(s) of the present invention or a H11 fusion protein encoded by a polynucleotide of the present invention. The term "vector" refers to a means, including, for example, a protein or a polynucleotide or a mixture thereof which is capable of being introduced or of introducing the proteins and/or polynucleotides of the invention into a cell. Certain vectors are particular suitable for the introduction of polynucleotides or polypeptides into only some specific cell types, while other vectors can be introduced into a variety of different cell types. The skilled artisan knows how to choose a particular vector depending on the cell type into which the polynucleotide or polypeptide is to be introduced.

In a preferred embodiment the vector of the present invention comprises plasmids; phagemids; phages; cosmids; artificial chromosomes, in particular artificial mammalian chromosomes or artificial yeast chromosomes; knock-out or knock-in constructs; viruses, in particular adenovirus, vaccinia virus, attenuated vaccinia virus, canary pox virus, lentivirus (Chang and Gay, 2001), herpes virus, in particular Herpes simplex virus (HSV-1, Carlezon, *et al.,* 2000), baculovirus, retrovirus, adeno-associated-virus (AAV, Carter and Samulski. 2000), rhinovirus, human immune deficiency virus (HIV), filovirus, and engineered versions of above mentioned viruses (see, for example, Kobinger *et al.,* 2001); virosomes; "naked" DNA, liposomes; virus-like particles; and nucleic acid coated particles, in particular gold spheres. Particularly preferred are viral vectors like adenoviral vectors, lentiviral vectors, baculovirus vectors or retroviral vectors (Lindemann *et al.,* 1997, and Springer *et al.,* 1998). Examples of plasmids, which allow the generation of such recombinant viral vectors include pFastBac1 (Invitrogen Corp., Carlsbad CA), pDCCMV (Wiznerowicz *et al.,* 1997) and pShuttle-CMV (Q-biogene, Carlsbad, California).

Liposomes are also preferred vectors and are usually small unilamellar or multilamellar vesicles made of cationic, neutral and/or anionic lipids, for example, by ultrasound treatment of liposomal suspensions. The polynucleotides can, for example, be ionically bound to the surface of the liposomes or internally enclosed in the liposome. Suitable lipid mixtures are known in the art and comprise, for example, DOTMA (1, 2-Dioleyloxpropyl-3-trimethylammoniumbromide) and DPOE (Dioleoylphosphatidyl-ethanolamine) which both have been used on a variety of cell lines.

Nucleic acid coated particles are another means for introducing the polynucleotides of the invention into cells using so called "gene guns", which allow the mechanical introduction of particles into the cells. Preferably the particles itself are inert, and therefore, are in a preferred embodiment made out of gold spheres.

In a further aspect the polynucleotide of the present invention is operatively linked to one or more expression control sequences, which allow expression in prokaryotic and/or eukaryotic host cells. The transcriptional/translational regulatory elements referred to above include, but are not limited to, inducible and non-inducible, constitutive, cell cycle regulated, metabolically regulated promoters, enhancers, operators, silencers, repressors and other elements that are known to those skilled in the art and that drive or otherwise regulate gene expression. Such regulatory elements include, but are not limited to, regulatory elements directing constitutive expression like, for example, promoters transcribed by RNA polymerase III like, e.g., promoters for the snRNA U6 or scRNA 7SK gene, the cytomegalovirus hCMV immediate early gene, the early or late promoters of SV40 adenovirus, viral promoter and activator sequences derived from, e.g., NBV, hepatits (HCV), herpes simplex virus (HSV), human papilloma virus (HPV), Ebstein-Barr virus (EBV), human T-cell leukaemia virus (HTLV), mouse mammary tumor virus (MMTV) or HIV; which allow inducible expression like, for example, CUP-1 promoter, the tet-repressor as employed, for example, in the tet-on or tet-off systems, the lac system, the trp system; regulatory elements directing cell cycle specific expression like, for example, cdc2, cdc25C or cyclin A promotor; or the TAC system, the TRC system, the major operator and promoter regions of phage A, the control regions of fd coat protein, the promoter for 3-phosphoglycerate kinase (PGK), the promoters of acid phosphatase, and the promoters of the yeast α- or a-mating factors. Particularly preferred promoters are the constitutive CMV immediate early gene promoter, the early or late SV 40 promoter, the polyhedrin promoter, retroviral LTRs, PGK promoter, elongation factor 1-α (EF1-α.) and phosphoenolpyruvate carboxy kinase (PEPCK).

As used herein, "operatively linked" means incorporated into a genetic construct so that expression control sequences effectively control expression of a coding sequence of interest.

Another aspect of the present invention is a host cell genetically engineered with the polynucleotide(s) or the vector(s) of the present invention outlined above. The host cells that may be used for purposes of the invention include, but are not limited to, prokaryotic cells such as bacteria (for example, *E. coli* and *B. subtilis),* which can be transformed with, for example, recombinant bacteriophage DNA, plasmid DNA, or cosmid DNA expression vectors containing the polynucleotide molecules of the invention; simple eukaryotic cells like yeast (for example, *Saccharomyces* and *Pichia),* which can be transformed with, for example, recombinant yeast expression vectors containing the polynucleotide molecule of the invention; insect cell systems like, for example, *Spodoptera frugiperda* and *Trichoplusioa ni* cell lines, e.g. Sf9 or Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing a H11 polypeptide encoding nucleotide sequence; or mammalian cell systems (for example, COS, CHO, BHK, HEK293, VERO, HeLa, MDCK, Wi38, NSO and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter and the vaccinia virus 7.5K promoter) or from bacterial cells (for example, the tet-repressor binding its employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells. Preferred host cells are *Spodoptera frugiperda, Trichoplusioa ni;* mammalian cells, in particular stem cells, hemopoietic cells, hepatocytes, adipocytes, neurons, osteoclasts, uterine endometrium cells, dermatocytes, myocardial cells, mucosal cells, leucocytes or tumor cells; bacterial cells, in particular of *Escherichia* or *Bacillus* species and yeast cells, in particular of *Pischia* or *Saccharomyces* species.

It has been discovered by the present inventors that tumor cells or cell lines, which are engineered with the polynucleotide(s) or the vector(s) of the present invention can be used to prevent or treat a variety of proliferative diseases. The tumor cells or cell lines can be derived from a wide variety of tumors and species. The species from which the tumor cell can be derived is preferably a mammal, selected from the group of human, non-human primate, horse, bovine, sheep, goat, pig, dog, cat, goat, rabbit, mouse, rat, guinea pig, hamster, or gerbil, in particular human. The tumor cell which is engineered can be derived directly from a tumor or can be further subcultured prior to engineering. The prolonged subculturing of a tumor cell will usually result in the establishment of a cell line, which primarily consists of clonal cells. The tumor cell or cell line can be derived from any tumor however, tumor cells derived from a cancer of the gastrointestinal or colorectal tract, liver, pancreas, kidney, bladder, prostate, endometrium, ovary, testes, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, glioma, astrocytoma, osteosarcoma, soft tissue sarcoma, hemangioma, endocrinological tumor, hematologic neoplasia including leukemia, lymphoma, and other myeloproliferative and lymphoproliferative diseases, carcinoma in situ, hyperplastic lesion, adenoma, fibroma, histiocytosis, chronic inflammatory proliferative disease, vascular proliferative disease and virus-induced proliferative disease are preferred. Particular preferred are tumor cells or cell lines derived from melanoma, pancreatic cancer and renal cancer. It is preferred that the tumor cell line, which is engineered with the polynucleotide(s) or vector(s) of the present invention and which is later used to prevent or treat a proliferative disease in one species has been derived from the same species (allogeneic origin) or even from the very subject that is treated (autologous origin). Thus, in a preferred embodiment human tumor cells or human tumor cell lines derived from a patient to be treated or from another patient having the same or similar tumor type are engineered with the polynucleotide or the vector of the invention and used to prevent or treat a proliferative disease in a human patient. Preferred tumor cell lines, which can be engineered include, but are not limited to, NSO, B78H1, Renca, Hep G2, B9, human melanoma, renal cancer, in particular renal cell carcinoma, pancreatic cancer, autologous and allogeneic T-cells.

Since one preferred aspect of the invention involves the transfer of the polynucleotides of the invention into certain viral vectors, which are convenient tools for generating cells engineered with the polynucleotides of the present invention, i.e. simply by infection, it is also envisioned that the host cells engineered with the polynucleotides of the present invention are cells capable of packaging the polynucleotide(s) of the present invention into virus. Thus, another preferred type of host cells are packaging cells, in particular amphotropic packaging cells like, for example, PA 317, ecotropic packaging cells like GP+E86, embryonic human renal cells - 239. PA 317 is suitably paired with the vector DCCMV comprising the polynucleotides of the invention to produce recombinant retrovirus particles containing H11 sequence.

It has been further observed by the present inventors that the *in vivo* anti tumor effect exerted by host cells, in particular tumor cells or cell lines, engineered with the polynucleotide(s) or vector(s) of the present invention can be further enhanced, if the cell is engineered to comprise at least one further polynucleotide. Thus in a preferred embodiment the host cell of the present invention is engineered with at least one further polynucleotide encoding at least one further polypeptide. This is preferentially achieved by using a vector, in particular one of the vectors indicated above with respect of H11 and the subsequent or simultaneously introduction of this(ese) vector(s) into the host cell. The one or more additional polynucleotide can be comprised in a separate vector or can be comprised within the same vector as the H11 encoding polynucleotide. It is preferred that the host cells simultaneously express both the H11 protein and the at least one further protein encoded by the at least one further polynucleotide. In a preferred embodiment the at least one further polynucleotide introduced into the host cell encodes a cytokine, in particular GM-CSF, IL-6, IL-11, IL-15, anti-TGF, EPO, interferons, LIF, OSM, CNTF, CT-1 and sIL-6 R/IL-6 fusion proteins, in particular Hyper-IL-6. In this context it is also preferred that the host cell is selected from one of the preferred host cells indicated above in relation to H11, i.e. tumor cells or cell lines.

Consequently, it is in particular preferred that the host cell expressing at least one H11 polypeptide of the present invention and at least one further polypeptide, in particular a cytokine (e.g. GM-CSF, IL-6 or IL-11) is derived from a cell or cell line originating from tissue involved in a proliferative disease, preferably a cancer of the gastrointestinal or colorectal tract, liver, pancreas, kidney, bladder, prostate, endometrium, ovary, testes, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancie including neuroblastoma, glioma, astrocytoma, osteosarcoma, soft tissue sarcoma, hemangioma, endocrinological tumor, hematologic neoplasia, including leukemia, lymphoma, and other myeloproliferative and lymphoproliferative diseases, carcinomas in situ, hyperplastic lesions, adenoma, fibroma, histiocytosis, chronic inflammatory proliferative disease, vascular proliferative disease and virus-induced proliferative disease. Particular preferred are tumor cells or cell lines derived from melanoma, pancreatic or renal cancer. Again this tumor cell or cell line can be autologous, allogeneic or xenogeneic.

A further aspect of the invention is process for producing cells capable of expressing H11. This process comprises genetically engineering cells *in vitro* with at least one vector of the present invention wherein said H11 is (are) encoded by the polynucleotide(s) of the present invention. The type of cell, which can be transformed is not limited and depends on the respective vector or vector system used to genetically engineer the cells. Vectors and vector systems, which are preferred for the transformation of certain cell types, have been indicated above. In addition it is preferred that the particular cells and cell lines outlined above are employed in this process of the invention.

A further aspect of the invention is a process for producing a H11 polypeptide encoded by the polynucleotide of the invention comprising: culturing the host cell of the present invention and recovering the H11 polypeptide encoded by said polynucleotide. The skilled practitioner is aware of a variety of expression systems, which yield high level expression of heterologous proteins and which can, thus, be used for production of the H11 polypeptide of the present invention. The choice of expression system depends on the required amount of protein and the required modifications. While it is standard to use single cell organisms for the expression of heterologous proteins, it is also possible to use cells derived from multicellular organisms. In principle, any such cell culture is workable, whether from vertebrate or invertebrate culture. In addition to mammalian cells, these include insect cell systems infected with recombinant virus expression vectors (e.g., baculovirus); plant cell systems infected with recombinant virus expression vectors (e.g., cauliflower mosaic virus, CaMV; tobacco mosaic virus, TMV); or transformed with recombinant plasmid expression vectors (e.g., Ti plasmid), yeast transformed with plasmids or artificial chromosomes and prokaryotic cells transformed with plasmids, cosmids, phagemids or phage containing one or more H11 polypeptide coding sequences.

In a useful insect system, *Autograph californica* nuclear polyhedrosis virus (AcNPV) is used as a vector to express foreign genes. The virus grows in *Spodoptera frugiperda* and *Trichoplusion ni* cells. *Spodopteria frugiperda* cells were used for amplification of the virus, while for H11 production H5 cells isolated from *Trichoplusia ni* were employed. The H11 polypeptide coding sequences are cloned into non-essential regions (for example, the polyhedrin gene) of the virus and placed under control of an AcNPV promoter (for example, the polyhedrin promoter).

Successful insertion of the coding sequences results in the inactivation of the polyhedrin gene and production of non-occluded recombinant virus (i.e., virus lacking the proteinaceous coat coded for by the polyhedrin gene). These recombinant viruses are then used to infect *Spodoptera frugiperda* cells in which the inserted gene is expressed (e.g., U. S. Patent No. 4,215,051, Smith, incorporated herein by reference). Examples of vectors which can be used to generate recombinant virus have been indicated above.

Examples of useful mammalian host cell lines are VERO and HeLa cells, CHO cell lines, WI 38, BHK, COS-7, 293, HepG2, NIH3T3, RIN, NSO and MDCK cell lines. In addition, a host cell strain may be chosen that modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the posttranslational processing and modification of proteins. Appropriate cell lines or host systems can be chosen to ensure the correct modification and processing of the foreign protein expressed.

Expression vectors for use in mammalian cells ordinarily include an origin of replication (as necessary), a promoter located in front of the gene to be expressed, along with any necessary ribosome binding sites, RNA splice sites, polyadenylation site, and transcriptional terminator sequences. The origin of replication may be provided either by construction of the vector to include an exogenous origin, such as may be derived from SV40 or other viral (e. g., Polyoma. Adeno, CMV, VSV, BPV) source, or may be provided by the host cell chromosomal replication mechanism. If the vector is integrated into the host cell chromosome, the latter is often sufficient.

The promoters may be derived from the genome of mammalian cells (e. g., metallothionein promoter) or from mammalian viruses (e.g., the adenovirus late promoter; the vaccinia virus 7.5K promoter). Further, it is also possible, and may be desirable, to utilize promoter or control sequences normally associated with the H11 polynucleotide provided such control sequences are compatible with the host cell system used.

A number of viral based expression systems may be utilized, for example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication. Smaller or larger SV40 fragments may also be used, provided there is included the approximately 250 bp sequence extending from the *Hind*III site toward the *Bgl*II site located in the viral origin of replication.

In cases where an adenovirus is used as an expression vector, the coding sequences may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (e.g., region E1, E3, or E4) will result in a recombinant virus that is viable and capable of expressing H11 fusion polypeptides in infected hosts.

Specific initiation signals may also be required for efficient translation of H11 fusion polypeptide coding sequences. These signals include the ATG initiation codon and adjacent sequences. Exogenous translational control signals, including the ATG initiation codon, may additionally need to be provided. One of ordinary skill in the art would readily be capable of determining this and providing the necessary signals. It is well known that the initiation codon must be in-frame (or in-phase) with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements and transcription terminators. In eukaryotic expression, one will also typically desire to incorporate into the transcriptional unit an appropriate polyadenylation site (e.g., 5'-AATAAA-3') if one was not contained within the original cloned segment. Typically, the poly A addition site is placed about 30 to 2000 nucleotides "downstream" of the termination site of the protein at a position prior to transcription termination.

For long-term, high-yield production of a recombinant fusion polypeptides stable expression is preferred. For example, cell lines that stably express constructs encoding a H11 fusion polypeptide may be engineered. Rather than using expression vectors that contain viral origins of replication, host cells can be transformed with vectors controlled by appropriate expression control elements (e.g., promoter, enhancer, sequences, transcription terminators, polyadenylation sites, etc.), and a selectable marker. Following the introduction of foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines.

A number of selection systems may be used including, but not limited to, the herpes simplex virus thymidine kinase (tk), hypoxanthine-guanine phosphoribosyltransferase (hgprt) and adenine phosphoribosyltransferase (aprt) genes, in tk-. hgprt-or aprt-cells, respectively. Also. antimetabolite resistance can be used as the basis of selection for dihydrofolate reductase (dhfr), that confers resistance to methotrexate; gpt, that confers resistance to mycophenolic acid; neomycin (neo), that confers resistance to the aminoglycoside G-418; and hygromycin (hygro), that confers resistance to hygromycin.

Animal cells can be propagated *in vitro* in two modes: as non-anchorage dependent cells growing in suspension throughout the bulk of the culture or as anchorage-dependent cells requiring attachment to a solid substrate for their propagation (i.e., a monolayer type of cell growth).

Non-anchorage dependent or suspension cultures from continuous established cell lines are the most widely used means of large scale production of cells and cell products. However, suspension cultured cells have limitations, such as tumorigenic potential and lower protein production than adherent cells.

Large scale suspension culture of mammalian cells in stirred tanks is a common method for production of recombinant proteins. Two suspension culture reactor designs are in wide use - the stirred reactor and the airlift reactor. The stirred design has successfully been used on an 8000 liter capacity for the production of interferon. Cells are grown in a stainless steel tank with a height-to-diameter ratio of 1: 1 to 3: 1. The culture is usually mixed with one or more agitators, based on bladed disks or marine propeller patterns. Agitator systems offering less shear forces than blades have been described. Agitation may be driven either directly or indirectly by magnetically coupled drives. Indirect drives reduce the risk of microbial contamination through seals on stirrer shafts.

The airlift reactor, also initially described for microbial fermentation and later adapted for mammalian culture, relies on a gas stream to both mix and oxygenate the culture. The gas stream enters a riser section of the reactor and drives circulation. The gas disengages at the culture surface, causing denser liquid free of gas bubbles to travel downward in the downcomer section of the reactor. The main advantage of this design is the simplicity and lack of need for mechanical mixing. Typically, the height-to-diameter ratio is 10: 1. The airlift reactor scales up relatively easy, has good mass transfer of gases and generates relatively low shear forces.

The H11 fusion polypeptide of the invention can be secreted from the cell into the supernatant or it can be maintained within the cell. While the cell supernatant can be directly submitted to further purification steps as outlined below, it is necessary to make protein within a cell accessible to further purification. Various methods are available to disrupt cells including chemical, e.g. chaotrophic agents (e.g. urea, sodium thiocyanate, GuHCl), high salt and/or detergents and mechanic means, e.g. French presses, freeze-thawing and/or sonification. To obtain essentially correctly folded non-denatured proteins, which is preferred in the context of the present invention, it is preferred to employ non denaturing methods like, for example, a combination of freeze-thawing and French pressing under mild detergent conditions.

The thus solubilized proteins or proteins comprised in the supernatant can be submitted to further purification steps, which are well known in the art and include without limitation the use of precipitation, chromatographic and affinity purification steps. The chromatographic methods which can be employed include ion exchange chromatography, size exclusion chromatography, dye adsorption etc. Affinity purification methods can use resin coated with IL-11, IL-11 R, sIL-11 R or parts thereof, antibodies specific to either IL-11, sIL-11 R or a tag which is comprised within the H11 fusion polypeptide or tag, comprised within the H11 fusion polypeptide, which specifically interact with another compound or resin. Examples of such tags are 6xHis, FLAG, myc-tag, chitin-tag, glutathione-S-transferase-tag etc. Preferably the tags are comprised at the C-and/or N-terminus of the H11 fusion polypeptide of the present invention, which allows the removal of the tag with, for example, an endopeptidase. Accordingly the final purified H11 protein preferably does not contain any tags, which might elicit an immune response once administered to a patient.

Another aspect of the invention is a H11 polypeptide having the amino acid sequence encoded by a polynucleotide of the invention or obtainable by the process mentioned above. The term "polypeptide" and "protein" are used interchangeably and refer to any peptide-linked chain of amino acids, regardless of length or posttranslational modification.

A further aspect of the present invention is a transgenic non-human animal containing a polynucleotide, a vector and/or a host cell as described above. The animal can be a mosaic animal, which means that only part of the cells making up the body comprise polynucleotides, vectors, and/or cells of the present invention or the animal can be a transgenic animal which means that all cells of the animal comprise the polynucleotides and/or vectors of the present invention or are derived from a cell of the present invention. Mosaic or transgenic animals can be either homo- or heterozygous with respect to the polynucleotides of the present invention contained in the cell. In a preferred embodiment the transgenic animals are either homo- or heterozygous knock-out or knock-in animals with respect to the genes which code for the proteins of the present invention. The animals can in principal be any animal, preferably, however, it is a mammal, selected from the group of non-human primate, horse, bovine, sheep, goat, pig, dog, cat, rabbit, mouse, rat, guinea pig, hamster, or gerbil.

Another aspect of the present invention is a process for producing a H11 encoded by a polynucleotide of the present invention comprising: culturing the host cell described above and recovering the polypeptide encoded by said polynucleotide. Preferred combinations of host cells and vectors are outlined above and further combination will be readily apparent to someone of skill in the art. Depending on the intended later use of the recovered peptide a suitable cell type can be chosen. Eukaryotic cells are preferably chosen, if it is desired that the proteins produced by the cells exhibit an essentially natural pattern of glycosylation and prokaryotic cells are chosen, if, for example, glycosylation or other modifications, which are normally introduced into proteins only in eukaryotic cells, are not desired or not needed.

To detect the expression of the fusion protein of the present invention it is possible to use most antibodies raised either against sIL-11 R or IL-11. However, to distinguish between the expression of sIL-11 R, IL-11 and the fusion protein formed thereof, it is in some aspects of the invention desirable to have an antibody, which specifically detects the fusion protein, but which shows only a weak or essentially no specificity to sIL-11 R and IL-11 alone. Thus in a further aspect the present invention relates to an antibody specific to the polypeptide encoded by the polynucleotide of the present invention or obtainable by the process of the present invention for producing the H11 polypeptide, which is essentially non-specific to sIL-11 R and IL-11. It is known in the art how to generate antibodies which only recognize a particular epitope within a protein. It is possible, for example, to immunize mammals, in particular mice or rabbits with a peptide that comprises the junction between the sIL-11 R and the IL-11. This will lead to antibodies, which specifically recognize the fusion protein. From thus produced antibodies those will be further selected, which show only a weak or essentially no specificity towards IL-11 and sIL-11R.

As outlined above the H11 polypeptide of the present invention can be used as such or in the context of a host cell, i.e. can be transfected into a host cell. In both embodiments the H11 polypeptide as such or the host cells can be used alone or in combination with additional substances and, therefore, the present invention in a further aspect relates to a pharmaceutical composition comprising at least one polypeptide and/or at least one host cell of the present invention and at least one further component selected from the group consisting of liposomes, virosomes, microsphere, niosomes, dendrimeres, stabilizers, buffers, excipients, and additives.

Excipients which facilitate production and administration of the compositions of the present invention are the art known excipients and include, for example, alginates, calcium carbonate, dextrose, fructose, lactose, maltose, maltodextrin, and the like. Stabilizers are also known in the art and comprise, for example, α-tocopherol and various carbohydrates.

It has been observed by the present inventors that host cells expressing the H11 polypeptide of the present invention if administered to mice have a strong anti-tumor response. In addition it was shown that H11 on its own is a strong agonist of IL 11 R and can thus be employed in the treatment of diseases requiring IL-11 action for treatment or amelioration. Thus, a further aspect of the present invention is the use of the host cell of the present invention or host cells obtainable according to one of the processes of the present invention or the H11 polypeptide of the present invention for the production of a medicament for the treatment or prevention of a disease selected from the group consisting of a proliferative disease, a cytopathy, radiation damage, an IL-11 dependent inflammatory disorder, IL-11 dependent degenerative disorder and IL-11 dependent or mediated soft tissue disorder.

The H11 polypeptide and host cells expressing H11 polypeptide, in particular the host cells expressing H11 polypeptide, can be employed in the treatment and/or prevention of a wide variety of different proliferative disease, however, a preferred proliferative disease treatable or preventable according to the present invention is selected from the group consisting of cancer of the gastrointestinal or colorectal tract, liver, pancreas, kidney, bladder, prostate, endometrium, ovary, testes, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, glioma, astrocytoma, osteosarcoma, soft tissue sarcoma, hemangioma, endocrinological tumor, hematologic neoplasia including leukemia, lymphoma, and other myeloproliferative and lymphoproliferative diseases, carcinoma in situ, hyperplastic lesion, adenoma, fibroma, histiocytosis, chronic inflammatory proliferative disease, vascular proliferative disease and virus-induced proliferative disease; in particular melanoma, pancreatic and renal cancer. In particular in the context of the treatment of proliferative diseases it is envisionable that patients are immunized with a "cancer vaccine" prior to the development of any symptoms of a disease, i.e. receive a protective immunization, or after they have developed symptoms of the disease, i.e. receive a therapeutic vaccination.

Host cells expressing H11 and at least one further cytokine, in particular GM-CSF can provide in the context of certain tumors an even stronger *in vivo* anti-tumor response than cells expressing H11 only. Therefore, it is preferred that cells expressing H11 and at least one further cytokine are used for the production of a medicament to prevent or treat a proliferative disease.

The H11 polypeptide and host cells expressing H11, in particular the H11 polypeptide can be employed in the treatment of a wide variety of cytopenias, however, preferred cytopenias, which are treatable or preventable according to the use of the present invention are selected from the group consisting of thrombocytopenia, hematocytopenia, and pancytopenia.

A person or animal, which voluntarily or involuntary has been exposed to radiation, including among others x-ray, α, β and γ-radiation can depending on the duration and intensity of the radiation suffer severe localized or systemic toxicity. In particular in radiation therapy of various diseases, including proliferative diseases the toxicity associated with radiation therapy are often limiting the dose that can be applied for treatment and/or the frequency of radiation treatment. However, it is know that, for example, breast carcinoma have a 50% chance of local recurrence, if treated with daily irradiation (Barker *et al.,* 1980) and only a 20 to 27% chance of local recurrence, if treated twice daily (Fastenberg *et al.,* 1985). Therefore, it is desirable to find substances, which can ameliorate the consequences of involuntary radiation exposure as well as substances which would allow administering higher radiation doses at the same level of toxicity or the same radiation doses as currently used in radiation therapy at a lower level of toxicity. The present inventors have now surprisingly found that H11 polypeptide and/or host cells expressing H11, in particular the H11 polypeptide can be used to prevent and/or treat and/or ameliorate the effects of radiation, i.e. can act as a radiation protectant. To this end H11 polypeptide and/or host cells expressing H11 can be administered prior, during and/or after radiation exposure. In particular in the context of radiation therapy it is preferred that H11 is administered prior to the therapy and after the therapy.

Furthermore, the H11 polypeptide and/or host cells expressing H11, in particular the H11 polypeptide can be employed in the treatment of a wide variety of IL 11 dependent inflammatory disorders, however, preferred IL 11 dependent inflammatory disorders, which are treatable or preventable according to the use of the present invention are selected from the group consisting of liver failure; hepatitis; hepatopathy; sepsis; chemotherapy or radiation-induced tissue damage, in particular lung damage; inflammatory diseases, in particular inflammatory bowel disease, rheumatoid arthritis, inflammatory liver disease; mucositis; allergies; endometriosis; vasculitis; vascular disease associated with endothelial inflammation, in particular ischaemic heart diseases or peripheral vascular disease; and psoriasis.

Similarly preferred IL-11 dependent degenerative diseases treatable or preventable according to the use of the present invention are selected from the group consisting of degenerative CNS diseases, PNS diseases and osteoarthritis. IL-11 dependent or mediated soft tissue disorders treatable or preventable according to the use of the present invention are selected from the group consisting of obesity and idiopathic female infertility.

As the effect of the IL 11 R agonist of the present invention can be enhanced by the coadministration of one or more cytokines it is preferred to administer at least one further cytokine prior, simultaneously or subsequently to the administration of H11 or the host cell expressing H11 (and, if desired a cytokine).

It is known that IL-11 can stimulate the differentiation of cells. This has been shown, for example, for the differentiation of hippocampal neurons. Presently it is envisioned that autologous, allogeneic or xenogeneic stem cells, which have been partially differentiated are injected into patients, where they localize to the target tissues and undergo terminal differentiation. To facilitate and aid this differentiation process the present inventors envision to use the H11 fusion polypeptide of the present invention or the H11 fusion polypeptide obtainable according to the process of the present invention for the manufacture of a medicament for adjuvant therapy, during stem cell therapy. Many different applications of stem cell therapy have been discussed in the prior art examples of such stem cell therapy include, but are not limited to, therapy of Parkinson's disease and ADA. Similarly the H11 fusion polypeptide of the present invention or obtainable according to a process of the present invention can be used to stimulate *in vitro* differentiation of cells, in particular stem cells or precursor cells. Such differentiated cells can then be employed in therapy themselves like, for example, Parkinson's disease and ADA.

The H11 expressing cells, for example, H11 modified melanoma cells can be applied in a wide variety of dosaging and application schemes which are known in the art of cellular vaccination. The dosaging and application will be varied to elicit a sustained immune response against the target or the vaccine, e.g tumor and/or the tumor vaccine. The immune response of a patient in response to vaccination can be determined by any technique known to someone of skill in the art including, but not limited to, ELISA. In the therapy or prevention of proliferative diseases the H11 expressing cells, in particular H11 modified melanoma cells are applied a range of 1 x 10⁵ to 1 x 10¹⁰ cells per dose, preferably in a range of 1 x 10⁶ - 5 x 10⁸ cells per dose. The dose can be injected into the patient using any route known to the skilled person including, for example, an intramuscular (i.m.), an intradermal (i.d.), a subcutaneous route (s.c.), preferably it is injected via a s.c. route. A typical schedule of vaccination comprises two phases: induction and boost phase. In induction phase vaccine is typically administered several times over a short period of time, for example, between 2 and 12 times in between 2 days and two weeks intervals, preferably 4 to 10 times in one to two week(s) intervals, more preferably 6 to 8 times in two weeks intervals and then in boost phase once a month.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Brief Description of the Figures and Drawings.

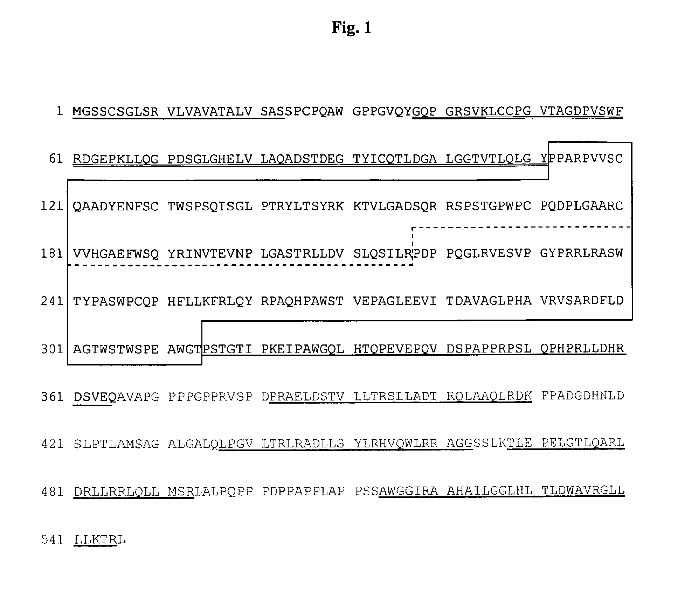

**Figure 1** displays amino acid sequence using the conventional one-letter code of the fusion protein H11 (SEQ ID NO 3). Sequence of sIL-11 R and IL-11 are indicated in black and grey, respectively. The different domains of the molecule are indicated as follow: N-terminal signal peptide (thin underline), the Ig-like domain (double underline), 200 AA hemopoietin domain composed of two 100 AA subdomains (D2 and D3) (the boxed region and subdomains are marked by broken line), the receptor pre-membrane region (line on the top of sequence), and IL-11 molecule where according to known three dimensional structure of CNTF the predicted α-helical regions are located and indicated by thick underline.

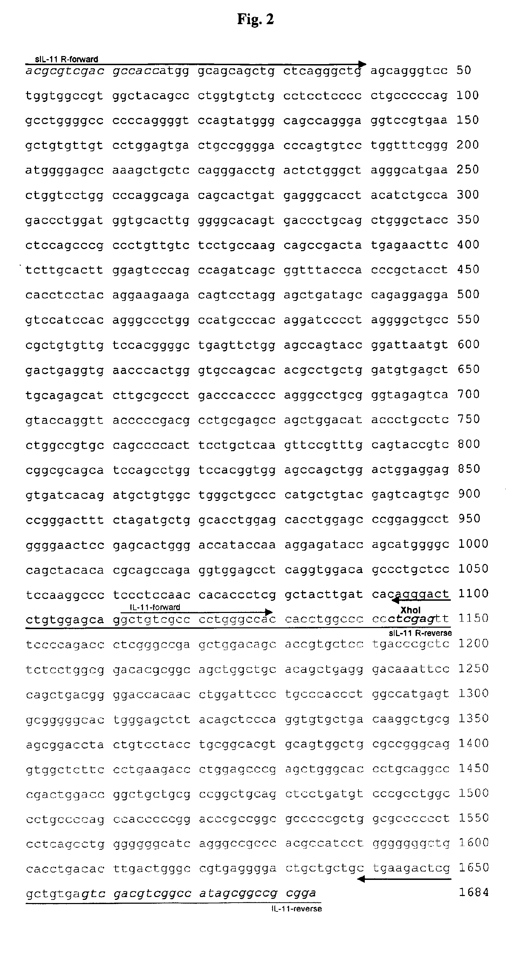

**Figure 2** shows a nucleic acid sequence of H11 (SEQ ID NO 6). The sequence of sIL-11 R and IL-11 are displayed in black and grey, respectively. Black arrows indicate sequence of primers used for amplification of sIL-11 R fragment and grey arrows the one used for IL-11. Arrows over and under the sequence represent the forward and reverse primers, respectively. Moreover, the restriction site XhoI, which was used to join both components is indicated.

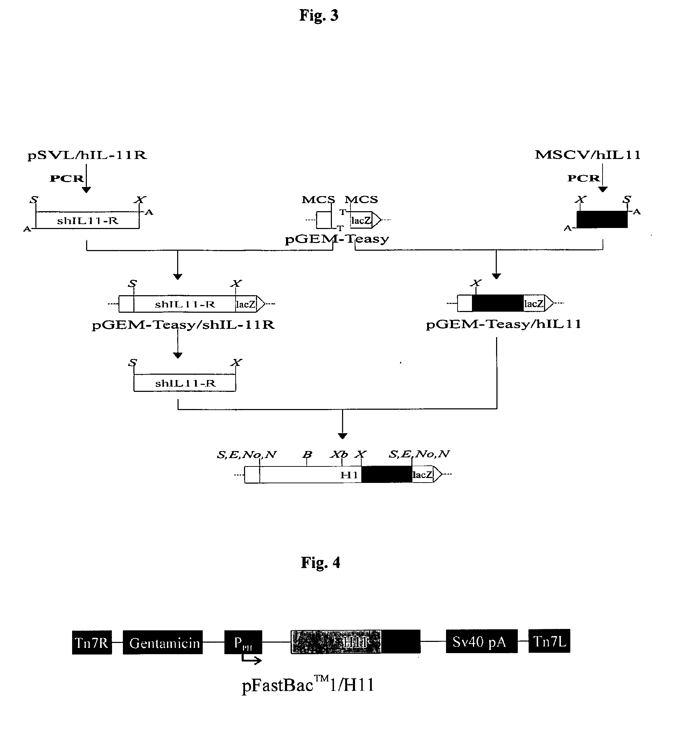

**Figure 3** is a schematic representation of the new designer cytokine construction steps as detailed in Example 1. Some restriction sites are indicated: S, X, E, No, N, B, Xb means SalI, XhoI, EcoRI, NcoI, NotI, BamHI, XbaI, respectively.

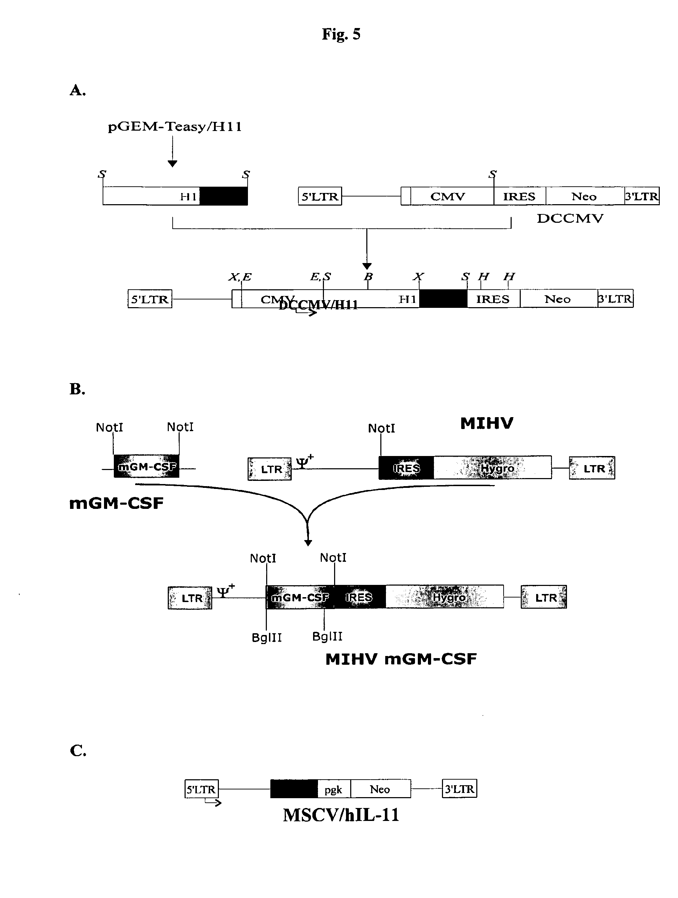

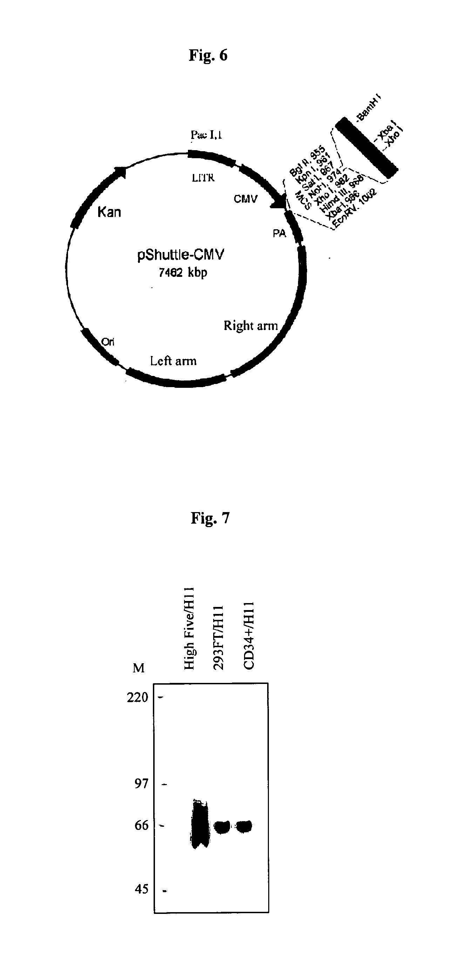

**Figures 4, 5 and 6** show schematic representations of vectors used for expression of recombinant protein in a Baculovirus expression system (Fig. 4. and Example 2.), for generating GMTV (Fig. 5., Example 3.) and recombinant transfer vector pShuttle-CMV/H11 for adenovirus construction (Fig. 6, Example 4).
**Figure 7** show an autoradiogram of a Western blot. Conditioned medium from transduced 293FT cells and CD34+ cells were electrophoresed (PAGE/SDS), transferred on PDV membrane and analyzed using anti-IL-11 and anti-IL-11R antibodies (Santa Cruz Biotechnology, Santa Cruz, CA) and goat anti-rabbit IgG/HRP as secondary antibody (DAKO, Glostrup, Denmark). Recombinant H11 collected from High Five cells infected with recombinant baculoviruses carrying H11 served as a positive control.

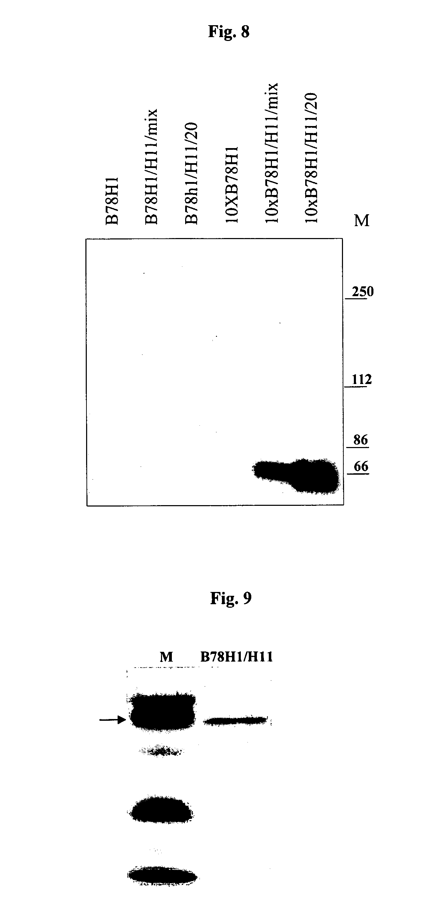

**Figures 8 and 9** show autoradiograms of Western blots. Conditioned media from transducted B78H1 cells were electrophoresed (PAGE/SDS), transferred on PDV membrane and analyzed using anti-IL-1-1 R antibody (Santa Cruz Biotechnology, Santa Cruz, CA) and goat anti-rabbit IgG/HRP as secondary antibody (Dako, Glostrup, Denmark). The analysis indicated that the fusion protein was properly expressed and secreted by tumor cells modified with recombinant retro- and adenoviruses (Fig. 8 and 9, respectively, Example 6).

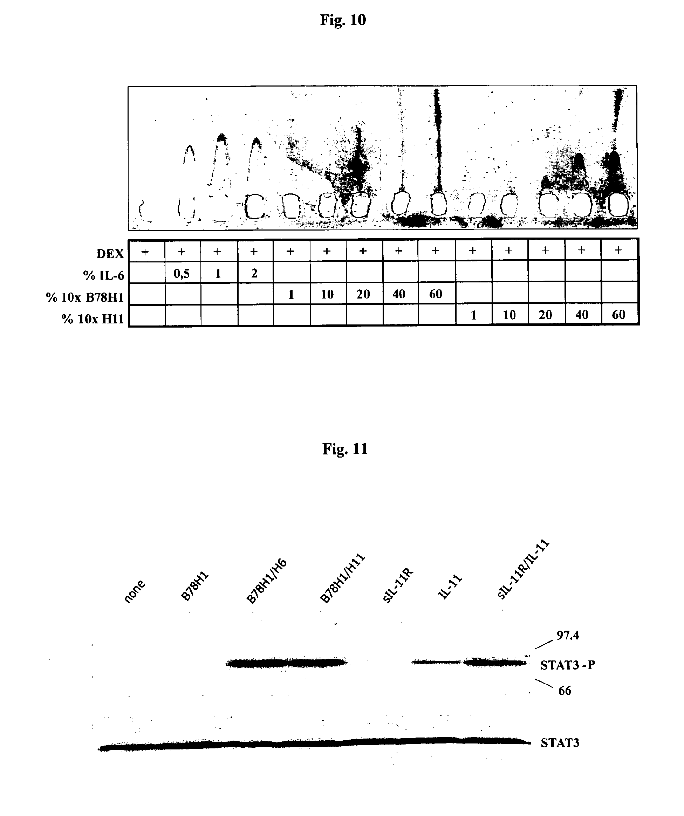

**Figure 10** is a photograph of rocket immunoelectrophoresis (Example 7). Briefly, HepG2 cells were stimulated with recombinant IL-6 and conditioned medium from control and H11 modified B78H1 cells at various concentrations (percentage of collected medium). After 48 h medium collected from stimulated HepG2 cells was analyzed by rocket immunoelectrophoresis for presence of α 1-antichymotrypsin.
**Figure 11** is an autoradiograph of the Western blot, where activation of STAT3 molecule was analyzed. Briefly, 3x10⁶ B9 cells were incubated in the presence of different cytokines: Hyper IL-6, H11 (50% of conditioned medium was collected from modified B78H1 cells with cDNA of Hyper IL-6 and H11, respectively), recombinant IL-11 (0,6 ng/ml), recombinant sIL-11 R (100 ng/ml) and complex of recombinant IL-11/sIL-11 R (0,6/100 ng/ml). After 30 minutes cells were lysed and proteins were electrophoresed (PAGE/SDS), transferred on PDV membrane and analyzed using anti-STAT3-P antibody (New England BioLabs, Beverly, MA) and secondary antibody goat anti-rabbit IgG/HRP (Dako, Glostrup, Denmark). As a negative control 50% conditioned medium from non-modified B78H1 cells was used.

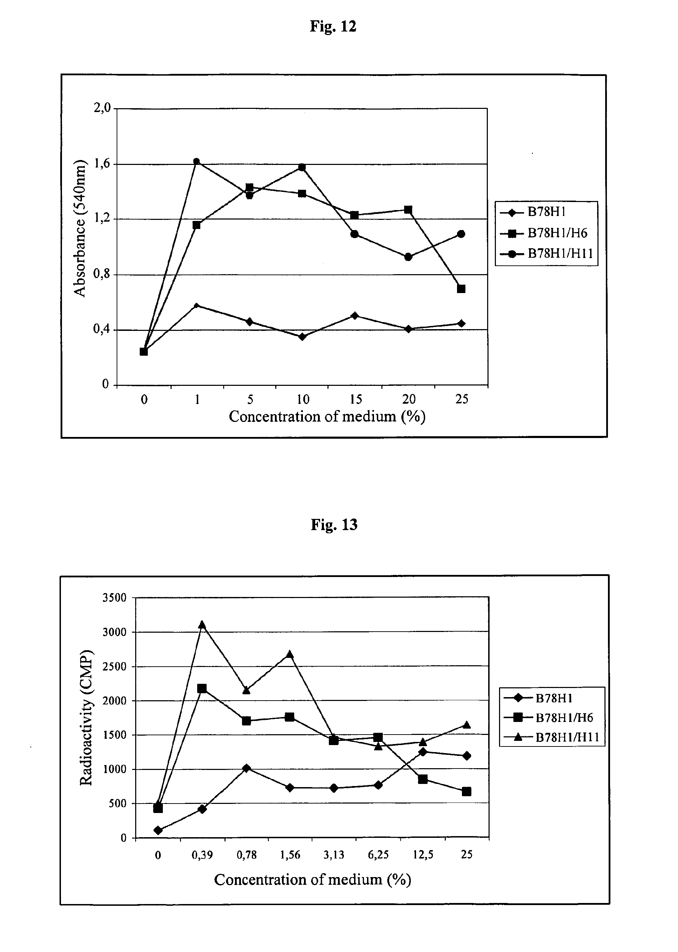

**Figures 12 and 13** are graphic representations of results from the proliferation assay of B9 cells. Briefly, B9 cells (2x10⁴ cells per well) were tested for proliferation at various concentrations (ranging from 1 to 25%) of medium collected from B78H1, B78H1/Hyper IL-6, B78H1/H11 cells. In the case, when medium collected after transduction of B78H1 cells with recombinant retroviruses carrying H11 was used, after 4 days of incubation test MMT (Sigma-Aldrich Corporation, St. Louis, MI) was performed. The absorbance was measured at 540 nm (Fig. 12). Activity of H11 obtained after adenoviral transduction was measured by incorporation of radioactive thymidine by proliferating B9 cells ([methyl-³H] thymidine, Sigma-Aldrich Corporation, St. Louis, MI) (Fig. 13).

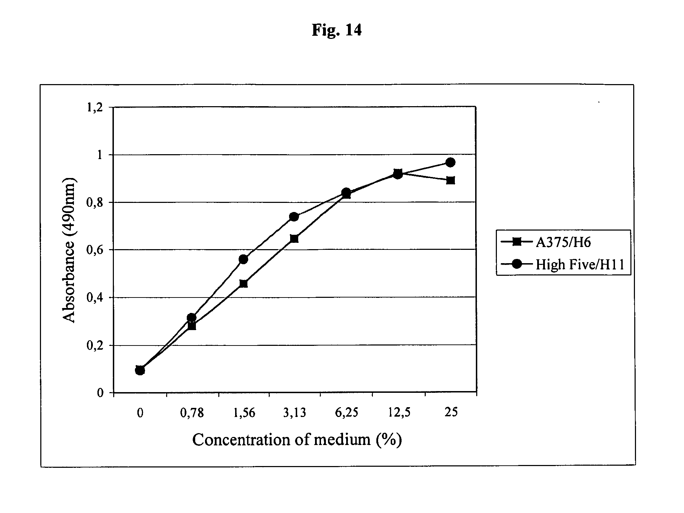

**Figure 14** is a graphic representation of results depicting the proliferation of Ba/Fgp130 cells induced by various concentrations of medium (ranging from 0,78 to 25%) collected from human melanoma A375 cells modified with Hyper IL-6 cDNA and recombinant H11 collected from cells infected with recombinant baculoviruses carrying H11 High-Five BTI-TN-5B1-4 (insect cells infected with recombinant baculoviruses carrying H11). The cell growth was quantified 3 days after seeding of 10x10⁴ Ba/Fgp130 cells per well. The number of living cells was measured by MMT test (Sigma-Aldrich Corporation, St. Louis, MI), which corresponded to the absorbance at 490 nm.

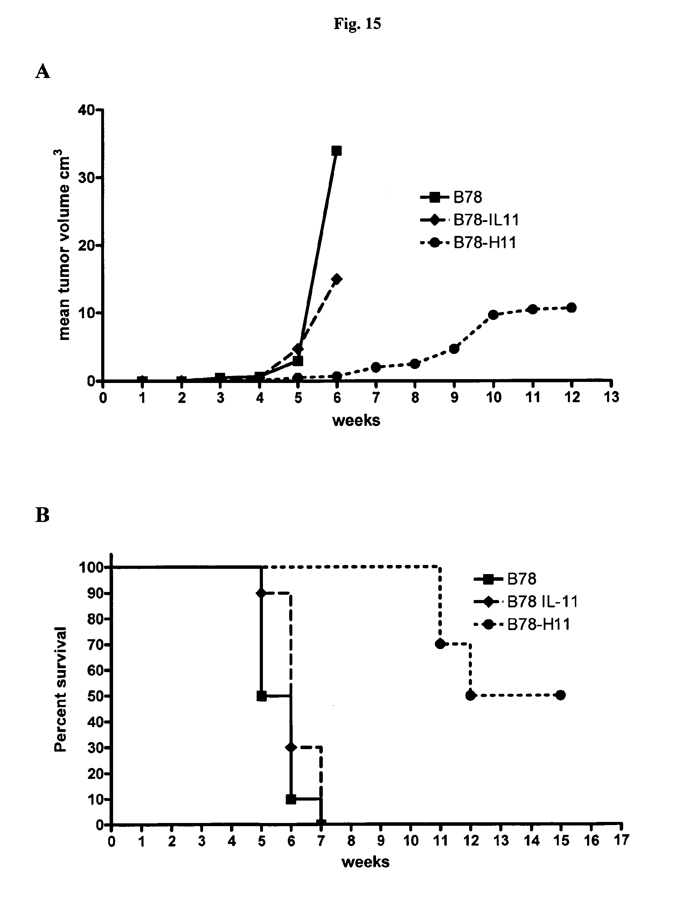

**Figure 15** is a graphic representation of results obtained from the analysis of *in vivo* activity of H11 in tumor rejection model. Briefly, C57BLxC3H mice (age 6-8 weeks) were injected subcutaneously (s.c.) with mock transduced and modified with cDNA of IL-11 and H11 murine melanoma cells B78H1 (5x10⁵ cells per mouse suspended in 0.1 ml PBS). Next, kinetics of tumor growth (Panel A) and survival of mice (Panel B) were monitored.

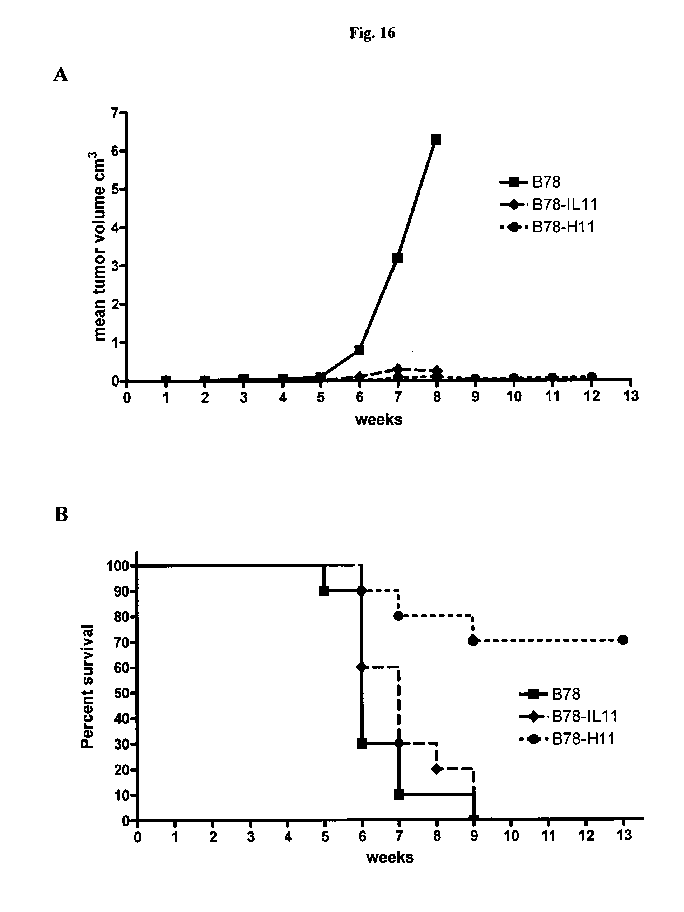

**Figure 16** is a graphic representation of results obtained after immunization of C57BLxC3H mice with control and modified B78H1 cells. C57BLxC3H mice were treated as indicated in description to Figure 15. After two weeks immunized mice were re-challenged s.c. with parental B78H1 cells (5x10⁵ cells per mouse) at a site distant from previous injection. Next, kinetics of tumor growth (Panel A) and survival of animals (Panel B) were monitored.

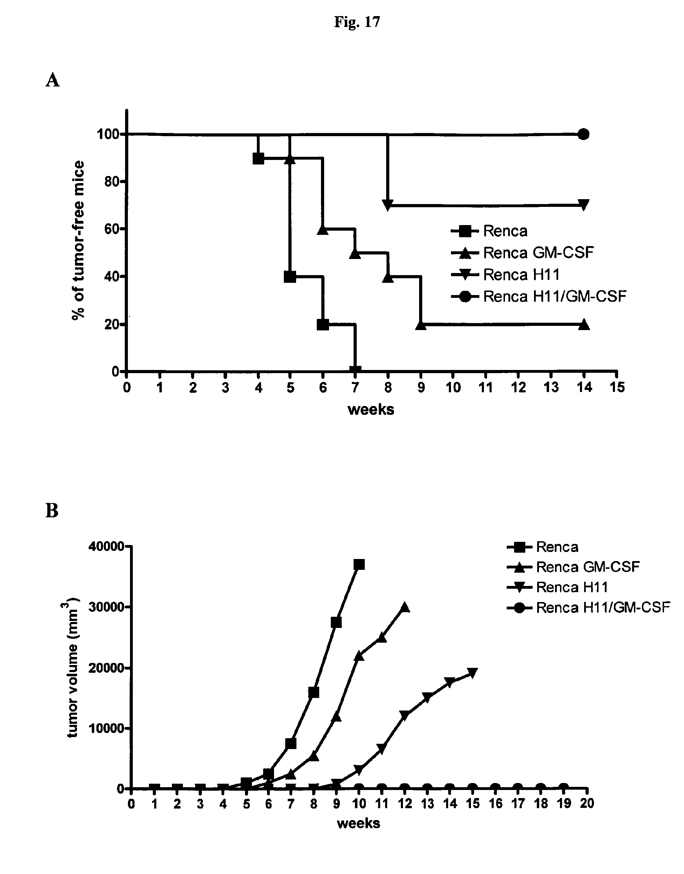

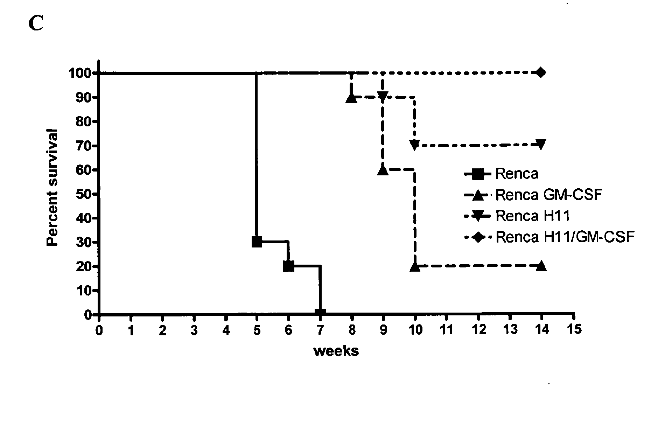

**Figure 17** is a graphic representation of analysis of modified tumor vaccine efficacy in tumor rejection model of renal carcinoma. Female Balb/c mice (age 8-12 weeks) were immunized by s.c. injection at right leg with control and gene modified with cDNA of GM-CSF and H11 and H11/GM-CSF Renca cells (5x10⁵ cells per mouse). Two weeks later, mice were re-challenged with parental Renca cells by s.c. injection at left leg. Next, analysis of tumor appearance (Panel A), tumor growth kinetics (Panel B) and mice survival (Panel C) were monitored.

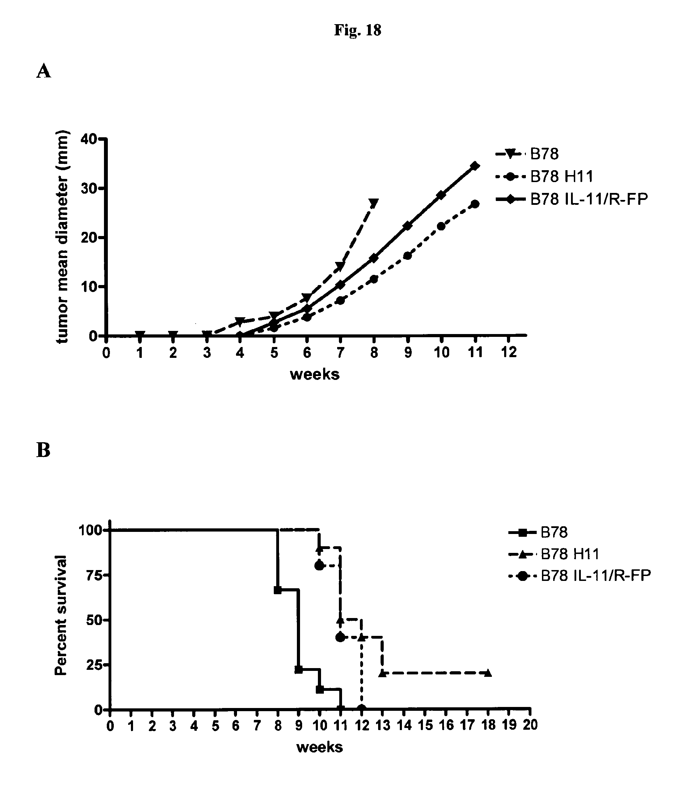

**Figure 18** depicts the tumorgenicity of B78-H1 melanoma cells modifed with H11 and IL-11/R-FP. C57BL6xC3H mice were injected with mock transduced and H-11 and IL-11/R-FP transduced B78-H1 cells. Tumor growth kinetics (Panel A) and mice survival (Panel B) were monitored once a week.

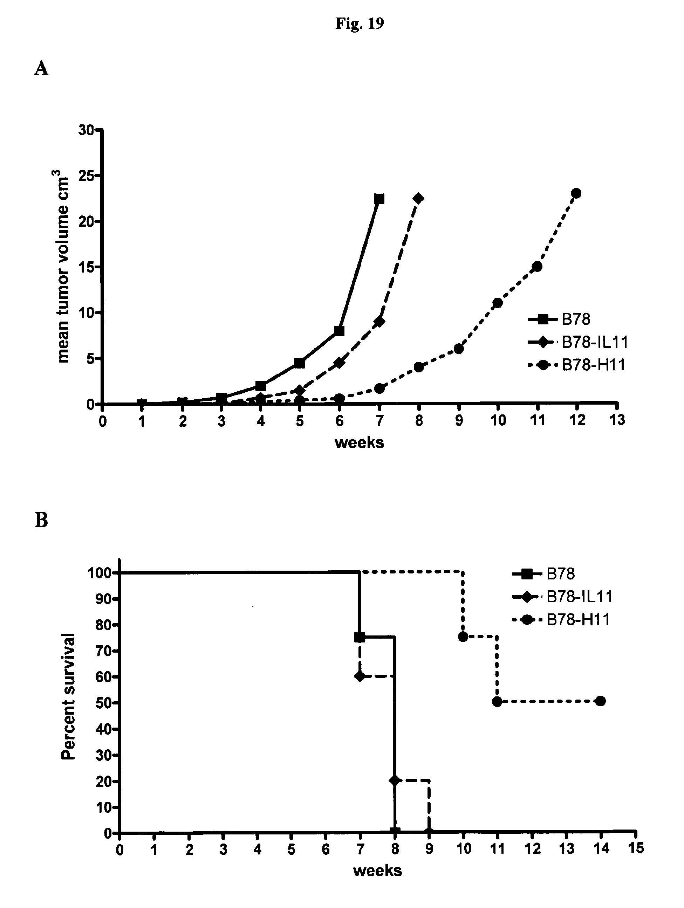

**Figure 19** is a graphic representation of study results of cellular mechanisms responsible for anti-melanoma activity of GMTV/H11. SCID mice (age 6-8 weeks) were s.c. injected with control and gene modified with cDNA of IL-11 and H11 B78H1 cells (5×10⁵ cells per mouse). Evaluation of the role of immunological cells in primary tumor rejection mediated by H11 was studied by analyses of tumor growth kinetics (Panel A) and SCID mice survival (Panel B).

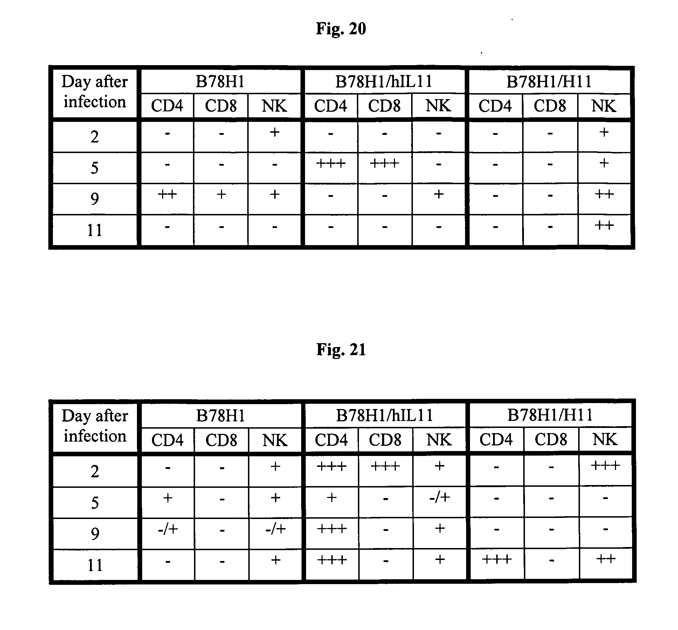

**Figures 20 and 21** summarize results obtained after immunostaining of CD4+, CD8+ and NK 1.1 of infiltrating cells at induction and effector phases of immune responses elicited by IL-11-and H11-GMTV. Briefly, two sets of experiments using C57BLxC3H mice were performed. One, in which mice were s.c. injected with control and IL-11 or H11 modified B78H1 cells (5x10⁵ cells per mouse) (Fig. 20), and second in which mice were injected as mentioned above and then two weeks later were re-challenged with parental B78H1 cells (5x10⁵ cells per mouse) (Fig. 21). At days 2, 5, 9, 11 following primary injection and after re-challenge mice were decapitated. Tissue from sites of injection was excised and frozen in liquid nitrogen. The 5µm thin cryosections were cut. For immunostaining sections were dried, fixed in cold acetone and then incubated overnight with primary antibody-biotin-conjugated rat anti-mouse CD4+ (PharMingen, San Diego, CA), biotin-conjugated rat anti-CD8+ (PharMingen, San Diego, CA) or mouse anti-mouse NK 1.1 cells (PharMingen, San Diego, CA). Slides were next incubated with biotin-conjugated rabbit anti-mouse IgG (Dako, Glostrup, Denmark) where needed. After blocking background peroxidase activity, sections were incubated in avidin-biotin peroxidase complex ABC/HRP (Dako, Glostrup, Denmark) and peroxidase substrate diaminobenzidine DAB (Dako, Glostrup, Denmark). Washed slides were counterstained with hematoxylin, dehydrated, mounted in Permount (Sigma-Aldrich Corporation, St. Louis, MI) and coverslipped. For assessment of the number of immunostained cells the following scale was used: "-"no stained cells, "-/+" bellow 5% of stained cells, "+" 5-20%, "++" 20-50% and "+++" above 50% of stained cells.

### EXAMPLES

For creation of H11 full length sIL-11 R amino acid sequence from M/1 - Q/365 was used. According to accumulated data it was possible to distinguish predicted regions: (i) signal peptide - position M/1 - S/23, (ii) Ig-like region - D1 domain, position G/38 - Y/111, (iii) CHD domain consisting of two FNIII regions - D2 and D3 domains, positions P/112 - R/217 and P/218 - T/314, respectively, and (iv) receptor pre-membrane region - part between the D3 domain and transmembrane region, position P/315 - Q/365 (Nandurkar *et al.,* 1996). In conformity with general idea, receptor is deprived of 26 and 31 amino acids of transmembrane and cytoplasmic regions, respectively.

Used sequence of IL-11 encodes full mature IL-11 protein (sequence A/19 - L/199), where all predicted regions: domains A, B, C, D and loops A-B, B-C, C-D could be distinguished. As mentioned above, IL-11 needs three specific binding sites (I, II, III) for interaction with its receptor. These sites are formed by combinations of amino acids from different domains and loops. Accordingly, the full length cytokine is necessary to display its activity. Both proteins (sIL-11 R and IL-11) have been connected on cDNA level by PCR/ligation reactions and no linker has been applied. In order to keep proper folding of the fusion molecule, the natural sequence of C-terminus of IL-11 R and N-terminus of IL-11 were used. The pre-membrane region of IL-11 R (approximately 50 amino acids) together with 16 N-terminal residues of IL-11 is not helical and presumably flexible, what can enable the proper assembly of the complex. The Figure 1 shows the amino acid (AA) sequence, where different domains of the molecule are indicated.

The new designer cytokine was constructed on cDNA level by standard PCR/ligation reactions (Example 1). The cDNA fragment of sIL-11 R (position 1-1095) was amplified using primers indicated in Example 1. The forward primer has additional sequence of restriction site *Sal*I*.* Moreover, ACC nucleotides in front of ATG and substitution A/G just after ATG were introduced in order to provide the Kozak consensus and restriction site *Nco*I*,* respectively. This mutation also led to the introduction of an amino acid substitution at position ²S 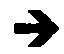 ²G. The reverse primer has additional sequence overlapping on 5' sequence of IL-11 to the place where restriction site *Xho*I occurs. The cDNA of IL-11 was amplified using primers as indicated in Example 1 (position 55-600). Amplified fragment omitted leader sequence of IL-11 with exception of natural sequence coding the last three amino acids (A V A) of the leader sequence which are now at the N termini of IL-11 component. The reverse primer contains an additional restriction site - *Sal*I*.* The amplified fragments were ligated according to Example 1. The sequence of H11 is shown in Figure 2 and steps of its construction are demonstrated in Figure 3.

As a next step the production of recombinant protein H11 in Baculovirus expression system was carried out (Example 2). The recombinant donor plasmid pFastBac1/H11 was constructed and recombinant bacmid was generated by site-specific transposition. Next, recombinant baculoviruses were propagated in Sf21 insect cells and used in an optimized system for production of fusion protein H11 in High-Five BTI-TN-5B1-4 insect cells. The purification of fusion protein was carried out by IEX and HIC chromatography as outlined below. Obtained protein was concentrated by membrane filtration and then used for *in vitro* study.

Moreover, retroviral vectors pDCCMV/H11, MIHV/GM-CSF and adenoviral transfer vector pShuttle-CMV/H11 were constructed (Example 3 and 4, respectively), which were used for modification of murine melanoma (B78H1) and renal carcinoma (Renca) cells (Example 6).

Modified cancer cells were able to express, process and secrete the fusion protein H11 (Fig. 8 and 9).

Medium collected from H11 modified cancer cells as well the purified recombinant H11 were tested for biological activity in three different *in vitro* bio-assays (Example 7). In human hepatoma cell line HepG2-assay, where sIL-11 R is able to induce production of acute phase proteins, in B9 (hybridoma cell line) bio-assay, where IL-11 induces cells proliferation, and in Ba/Fgp130 bio-assay where IL-11/sIL-11 R complex induces cell proliferation.

Moreover, *in vivo* activity of H11 was assessed using murine tumor rejection model (Example 8). H11 modification of murine melanoma, as well as renal carcinoma cells decreased the tumorgenicity and stimulated the long lasting anti-tumor immunity in mice. The modification of Renca cells with both H11 and GM-CSF was the most effective in this model (for a more detailed description see Example 8).

In order to assess cellular mechanisms responsible for anti cancer activity of H11 modified vaccine (GMTV/H11), two sets of experiments were performed (described in detail in Example 9). Using SCID mice we demonstrated that H11 activity against primary tumor might be associated with non-specific immunological reactions. Analyzing immunological cells infiltration (immunohistochemistry) we found that in induction phase of immune response neither CD4+ nor CD8+ cells were involved. However, we observed dense infiltration of NK cells, what confirmed the important role of these cells in growth inhibition of the primary tumor modified with H11. The major effector cells stimulated by H11/GMTV were NK calls and in the late phase CD4+ T lymphocytes.

### Example 1: Construction of a new designer cytokine Hyper IL-11 (H11).

The cDNA of human IL-11 (sequence position 55-600 which corresponds to the amino acid residues 19-199) has been amplified by standard PCR reaction using following primers:

IL-11forward: 5' GCT GCT GCC CCT GGG CCA (SEQ ID NO. 7),

IL-11reverse: 5' TCC GCG GCC GCT ATG GCC GAC GTC GAC TCA CAG CCG AGT CTT CAG
(SEQ ID NO. 8).

The amplified fragment of IL-11 was cloned into pGEM-Teasy vector (Promega, Madison, WI).

The cDNA of human IL-11 R (position 1-1095 which corresponds to the amino acid residues 1-365) has also been amplified by PCR reaction using forward primer containing a restriction site *Sal*I and special reverse primer with extra sequence overlapping on 5' sequence of IL-11 to the place where restriction site *Xho*I occurs. The sequences of used primers were:

sIL-11 R forward: 5' ACG CGT CGA CGC CAC CAT GGG CAG CAG CTG CTC AGG GCT G (SEQ
ID NO. 9)

sIL-11 R reverse: 5' AAC TCG AGG GGG GCC AGG TGG TGG CCC AGG GGC GAC AGC CTG
CTC CAC AGA GTC CCT (SEQ ID NO. 10).

The PCR product has been cloned into pGEM-Teasy vector and then digested with *Sal*I and *Xho*I restriction enzymes. The purified fragment *Sal*I*lXho*I was cloned into the *Xho*I site of the plasmid pGEM-Teasy/IL-11 leading to creation of the fusion cDNA of sIL-11 R and IL-11 (without any artificial linker sequence) referred to as Hyper IL-11 (H11).

The nucleotide sequence of new designer cytokine H11 is shown in Fig.2 and steps of its construction are demonstrated in Fig. 3.

### Example 2: Production of recombinant H11.

### Construction of recombinant donor plasmid (FastBac1/H11).

Plasmid pFastBac1 was used to generate viruses which express recombinant protein H11. pGEM-Teasy/H11 plasmid was digested with *Sal*I/*Sph*I restriction enzymes, purified fragment was then cloned into *Sal*I/*Sph*I restriction sites into pFastBac1 plasmid (Invitrogen Corporation, Carlsbad, CA) (Fig. 4).

### Generating a recombinant bacmid by site-specific transposition.

Generation of a recombinant baculovirus is based on site-specific transposition of an expression cassette into baculovirus shuttle vector (bacmid) propagated in E. coli.

The pFastBac1/H11 plasmid was transformed into DH10Bac competent cells which contain the bacmid with a mini-*att*Tn7 target site and the helper plasmid. The mini-Tn7 element on the pFastBac1 donor plasmid was transposed to the mini-attTn7 target site on the bacmid in the presence of transposition proteins provided by the helper plasmid. Colonies containing recombinant bacmids were identified by antibiotic selection and blue/white screening. Next, the high molecular weight mini-prep DNA was prepared. Isolated DNA was analyzed by PCR reaction for confirming the presence of inserted gene. The following primers were used:

Forward #9: 5' ACC CAC CCG CTA CCT CAC CT (SEQ ID NO 11)

Reverse #2: 5' GTC AGG AGC ACG GTG CT (SEQ ID NO 12)

### Transfection of Sf21 cells with recombinant bacmid DNA and propagation of the recombinant baculovirus.

The Sf21 *(Spodoptera frugiperda)* cells were transfected with recombinant bacmid DNA using Effectene Transfection Reagent (Qiagen, Valencia, CA). After 3 days supernatants were collected and titers of the virus were assessed. For amplifying viral stocks, 1.4x10⁶ of Sf21 cells suspended in Serum Free Medium (SFM) were infected at Multiplicity of Infection (MOI) 0.1 for 72 hours. The amplifying procedure of the recombinant H11 baculovirus was performed twice and finaly the high-quality, high- titer (2.87x10⁸ pfu/ml) master virus stock was obtained. The recombinant virus stock was stored at -80°C.

### Optimizing of heterologous protein production.

In order to achieve the optimal production of recombinant H11 protein, different factors were considered, such as cell lines (Sf21 and *Trichoplusia ni -* High-Five BTI-TN-5B1-4), medium (Sf900 II and Express5), parameters of viral infection (MOI) and expression kinetics. To express recombinant gene product, suspension cultures of High Five cells at a cell density 1, 1.5, and 2 x 10⁶ cells/ml were infected at MOI 0.5, 1, 5, and 10 pfu/ml and expression of H11 was monitored by Western blot analysis at different harvest time points. The optimized production of recombinant H11 protein was achieved at cell density of 1x10⁶ cells/ml at MOI 5 for 48 hours using High-Five BTI-TN-5B1-4 cells in Express5 (Invitrogen Corporation, Carlsbad, CA) medium. Moreover, production of H11 was performed in presence of Protease Inhibitor Cocktail for use in culture medium (Sigma-Aldrich Corporation, St. Louis, MI) at 1:1000 dilution.

### Purificaton of recombinant H11 protein.

Harvested supernatants were diluted with 20 mM 1,3 diaminopropane buffer pH 11.8 at ratio 1 : 8. Next, the precipitated proteins were removed by centrifugation at 8 000 g/4°C and batch IEX chromatography was performed overnight at 4°C using Q XL anion exchange medium (Amersham Pharmacia Biotech, Buckinghamshire, UK). Adsorbed proteins were regained in 0.5 M NaCl, 20 mM 1,3 diaminopropane buffer pH 10.5, concentration of NaCl was increased to 4 M and column HIC was preformed. The phenyl FF (low sub) medium (Amersham Pharmacia Biotech, Buckinghamshire, UK) was equilibrated with 4 M NaCl, 20 mM 1,3 diaminopropane buffer pH 10.5, loaded with proteins, washed with high molar start buffer and next eluted using linear descending gradient of salt concentration (4-0 M NaCl) in 20 mM 1,3 diaminopropane buffer pH 10.5. Fractions containing recombinant H11 protein were pooled and concentrated by membrane filtration using membrane with cut-off 30kDa (Millipore Corporation, Bedford, MA)

### Example 3: Construction of retroviral vectors DCCMV/H11 and MIHV/GM-CSF and generation recombinant retrovirus particles.

The cDNA of H11 was digested with NotI restriction enzyme and purified fragment was cloned into NotI site of dicistronic double-copy retroviral vector (DCCMV) (Wiznerowicz *et al.,* 1997). DCCMV's expression cassette placed in U3 region of 3'LTR contained CMV-IE promoter which drives the expression downstream H11 and Neo resistance genes. The steps of construction of DCCMV/H11 are shown in Fig. 5A.

Construction of MIHV/GM-CSF vector was performed by cloning of a murine GM-CSF cDNA excised by NotI digestion from pGEM-Teasy/mGM-CSF plasmid into NotI site of the MIHV retroviral vector (Fig. 5B)

The Fig. 5C shows retroviral vector MSCV/hIL-11 (Murine Stem Cell Virus) which was used as control in the study (gift from Dr. R Hawley, Toronto, Canada).

The DCCMV/H11 vector was transfected into amphotrophic packaging cell line (PA 317) via electroporation (250V/104ms). After 14 days of selection in the presence of antibiotic G418 (500µg/ml) supernatants containing recombinant retroviruses were collected and frozen at - 80°C. In order to produce recombinant retroviruses carrying hIL-11 and GM-CSF the procedure as described above was performed with exception of selection in Hygromycin (150µg/ml) when MIHV/GM-CSF vector was used.

### Example 4: Construction of adenoviral transfer vector pShuttle-CMV/H11 and generation of recombinant adenoviruses.

The cDNA of H11 digested with *Not*I/*Sal*I restriction enzymes was cloned into *Not*I/*Xho*I site of transfer plasmid pShuttle-CMV (Q-biogene, Carlsbad, CA) generating pShuttle-CMV/H11 (Fig. 6). Next, linearized with *Pme*I pShuttle-CMV/H11 plasmid was co-transformed into bacteria with plasmid carrying adenoviral genes pAdEasy1 (Q-biogene, Carlsbad, CA) where homologous recombination occurs. After selection in Kanamycin (50 µg/ml) the recombinant plasmid was isolated, linearized with *Pac*I and transfected into QBI-293A cells (Q-biogene, Carlsbad, CA) which provide in trans adenovirus proteins necessary to generate adenovirus particles. Next, the adenoviruses carrying H11 were amplified in QBI-293A cells, purified by discontinuous cesium chloride gradient, dialyzed in PBS and stored in -800°C. The titer of high-quality master virus stock was 1.25x10⁸ pfu/ml.

### Example 5: Construction of lentivirus expressing recombinant H11 protein.

To construct a lentivirus expressing recombinant H11 protein a retroviral system based on the human immunodeficiency virus (HIV) using three-plasmids, which was developed by Didier Trono (see, for example, Dull *et al.,* 1998 or Zufferey *et al.,* 1998) was used. Lentiviral vector particles are generated by transient transfection of vector-producing cell line with following plasmids: transducing vector, packaging vector and envelope vector. The transducing vector pWPXL contains a cis-acting sequences of HIV required for packaging, reverse transcription and integration, internal promoter and enhancers and unique restriction sites for cloning of cDNAs. Packaging construct pCMV-deltaR8.91 encodes Gag, Pol, Tat and rev viral proteins. The envelope vector pMD2G-VSVG expresses the surface glycoprotein (G) of VSV. pWPXL is a self-inactivating vector with a deletion in the 3' long terminal repeat (LTR), which abolished the LTR promoter activity.

pWPXL-EF1alpa-GFP plasmid was used to generate lentivirus which expresses recombinant protein H11 under control of EF1 alpha promoter. cDNA of GFP was excised with *Pme*I and *EcoR*I and the *Pme*I restriction site was filled in using DNA I polymerase Klenow Fragment to generate blunt ends. To obtain cDNA for H11 pGEM-Teasy/H11 plasmid was digested with *Spe*I*,* blunt ends were generated with DNA I polymerase Klenow Fragment, and then the plasmid was digested with *Eco*RI*.* The purified fragment of H11 was cloned into blunt end/*Eco*RI restriction sites of the plasmid pWPXL.

Production of lentivirus vectors expressing H11 LV-H11, was based on co-transfection of 293FT cell line (Invitrogen) with three plasmids pWPXL-H11, pCMV-deltaR8.91, pMD2G-VSVG. The 293FT cell line is derived from the 293F cell line and stably expresses the SV40 large T antigen. The 293F cell line is a fast-growing variant of 293 cell line - established from primary embryonic human kidney transformed with sheared human adenovirus type 5 DNA.

LV-H11 was used to transduce 293FT cell line and CD34+ primary human cells. Stable gene transfer was confirmed by the PCR method. Two sets of primers, corresponding to both IL-11 and sIL-11R parts of Hyper-IL-11, were used to amplify IL-11 and sIL-11R from genomic DNA isolated from transduced cells. Expression of H11 protein in 293FT cells and CD34+ was analyzed by Western blotting (Fig. 7).

### Example 6: Gene modification of murine cancer cells (B78H1 and Renca).

Collected supernatants containing recombinant retroviruses caring H11 were used for transduction of murine cancer cell: melanoma cell line (B78H1) and renal carcinoma cell line (Renca). The transduced cells were selected in G418 as previously described and then several clones have been isolated. The RNA from obtained clones was isolated according to standard Chomczynski and Sacchi (Chomczynski and Sacchi, 1987) procedure and expression of H11 was analyzed using Northern blot with cDNA of hIL-11 and hIL-11 R as specific probes. The mRNA for hIL-11 and hIL-11 R was found exactly at the same position indicating that both target sequences are on one mRNA. The Northern blot analysis permitted to select the clones of the highest level of H11 expression. Moreover, the supernatant from these clones was analyzed by Western blot using anti-IL-11 R antibody and demonstrated that H11 is secreted by the cells (Fig. 8).

For control study the B78H1 cells were transduced using MSCV/hIL-11 vector and Renca cells were modified with MIHV/GM-CSF vectors. Moreover, Renca/H11 cells were cotransduced with MIHV/GM-CSF and selected in Hygromycin (150 µg/ml). Proteins (IL-11 and GM-CSF) secreted *in vitro* into culture medium were measured by ELISA (RαD, Minneapolis, MN).

Moreover, B78H1 cells were transduced with adenoviruses carrying H11. After overnight incubation, medium was changed and two days later the presence of H1 in culture medium was confirmed by Western blot analysis (Fig. 9).

### Example 7: Assessment of H11 activity in vitro.

The recombinant H11 and supernatant from B78H1/H11 transduced cells were analyzed for biological activity in three different bio-assays: HepG2, B9 and Ba/Fgp130.

The HepG2 cells (human hepatoma cell line) secret endogenous IL-11, what makes them unresponsive to exogenous IL-11. However, HepG2 insensibility to IL-11 can be restored by addition of sIL-11 R. Stimulation of HepG2 cells with H11 but not with IL-11 caused increased secretion of α₁-antichymotripsin (measured by rocket immunoelectrophoresis) indicating that fusion protein H11 is biologically active *in vitro* (Fig. 10).

The B9 cells (hybridoma cell line) posses IL-11 R and gp130 receptors, what makes them responsive to IL-11. Stimulation of B9 cells with IL-11 and H11 (medium collected from transduced B78H1 cells with retro- and adenoviruses carrying H11) led to the signal transduction causing phosphorylation of STAT3 molecules (Fig. 11), what finally resulted in the proliferation of B9 cells (Fig. 12 and 13). These results further indicate that H11 fusion protein is active *in vitro.*

The unique feature of Ba/F cells (pro-B lymphocyte cell line) is the lack of membrane gp130 molecules, what makes them unresponsive to IL-11/sIL-11 R complex. However, transfection of Ba/F cells with gp130 cDNA makes them responsive to the combination of IL-11 and sIL-11 R. Stimulation of Ba/Fgp130 cells with recombinant H11 led to their propagation as measured using MTT test (Fig. 14).

Obtained results demonstrated that H11 protein produced in three different systems (Baculovirus Expression System, as well as protein excreted by eukaryotic cells transduced with retro- and adenoviruses) is active *in vitro.* Moreover, fusion protein is secreted from eukaryotic cells following transduction with retroviral and adenoviral vectors. The Ba/Fgp130 assay proved that the new designer cytokine H11 is acting as a complex.

### Example 8: In vivo anti-tumor activity of H11 in animal models.

In order to asses anti-melanoma activity of H11 the transduced B78H1 cells (5x10⁵) were injected into C57BLxC3H mice subcutaneously (s.c.) and the tumor growth and survival were monitored. As a control mock and IL-11 transduced cells were used.

The transduction of H11 into B78H1 cells inhibited tumor growth to the significantly higher level than IL-11 and control. Tumors appeared later and the mean volume of tumors in mice injected with B78H1/H11 cells was several fold smaller than in control groups (Fig. 15A). The overall survival of mice injected with B78H1/H11 cells was 7 weeks longer than mice injected with mock and IL-11 transduced B78H1 cells. Animals injected with B78H1 and B78H1/IL-11 cells survived maximally 7 weeks, while 50% of mice injected with B78H1/H11 cells survived 12 weeks (Fig. 15B).

In second series of experiments naive mice were primarily immunized s.c. with mock and B78H1 transduced cells and after two weeks re-challenged with parental B78H1 cells. The 90% of mice immunized with B78H1 cells developed tumors at 7^{th} week, while at the same time only 30% and 50% animals vaccinated with B78H1/H11 and B78H1/IL-11 respectively developed tumors. The mean volume of tumors in mice immunized with H11 modified vaccine was several fold smaller when compared to the control group (Fig. 16A). Mice vaccinated with control and IL-11 transduced vaccine survived 9 weeks, while 70% of animals immunized with H11 modified vaccine survived 12 weeks (Fig.16B).

Moreover, cancer vaccine modified with H-11 and GM-CSF genes has been analyzed in a tumor rejection model of a renal cell carcinoma (Renca cells). Female Balb/c mice at the age of 8 -12 weeks have been used. Four experimental groups were created, each consisted of 10 animals. Control mice received mock transduced Renca cells and other experimental groups received Renca cells expressing GM-CSF, H-11 or cells co-expressing H11 and GM-CSF, respectively. Immunization was performed by subcutaneous injection of mice with 5x10⁵ control or gene-modified cells, suspended in 0.125 ml PBS. Two weeks later, mice were challenged with parental Renca cells injected subcutaneously at a distant site. Evaluation of vaccine efficacy was based on analysis of tumor appearance, growth kinetics and survival of animals.

Mice immunized with unmodified cells started to develop tumors 4 weeks following the challenge, faster than other groups. All animals in the control group developed tumors within 7 weeks after administration of parental Renca cells. Mice immunized with Renca-GM-CSF started to develop tumors 5 weeks following the challenge. During the whole experiment 20% of mice in this group remained tumor-free. Immunization with Renca-H11 vaccine protected 70% of animals from the tumors and in 30% of mice tumors appeared later than in groups receiving control or GM-CSF-secreting Renca cells. Mice immunized with vaccine coexpressing H11 and GM-CSF genes completely rejected implanted parental tumor cells (Fig. 17A).

The most striking kinetics of tumor growth was observed in mice immunized with mock transduced Renca cells. Immunization with Renca-GM-CSF and -H11 vaccine decreased the tumor growth kinetics; however the Renca-H11 demonstrated a stronger protective effect than the Renca GM-CSF vaccine. No tumors were observed in the mice immunized with Renca H11/GM-CSF multigene vaccine (Fig. 17B).

Survival of mice immunized with control or gene-modified Renca cells was analyzed in a second set of experiments. The highest and fastest death rate was observed in mice immunized with control Renca cells. Immunization with Renca GM-CSF, H11 and H11/GM-CSF vaccine increased the survival of mice challenged with parental Renca cells. At the end of experiment 20, 70 and 100 % of mice, respectively were still alive (Fig. 17C).

### Example 9: In vivo anti-tumor activity of H11 compared to IL-11/R-FP.

In order to compare anti-tumor activity of H11 and IL-11/R-FP B78H1 cells were transduced with H11 or IL-11/R-FP cDNA and mock transduced modified cells were injected s.c. into mice and tumorogeneity was analyzed. H11 showed a better reaction in tumor growth than IL-11/R-FP (Fig. 18A and B).

### Example 10: Assessment of cellular mechanisms responsible for anti-melanoma activity of H11 modified vaccine (GMTV-H11).

In the first set of experiments SCID mice were used. They lack T and B lymphocytes, but possess NK cells, what allowed evaluation of the role of these cells in primary tumor rejection mediated by H11. In mice which received s.c. B78H1/IL-11 and B78H1/H11 cells tumors appeared week later than in control mice. The mean volume of tumors in B78H1/H11 cells injected mice was significantly smaller than in mice injected with mock and IL-11 transduced B78H1 cells (Fig. 19A). The survival of SCID mice vaccinated with B78H1/H11 cells was extended when compared to the control groups. Animals which received B78H1 and B78H1/IL-11 cells survived 8 and 9 weeks, respectively, while 50% of SCID mice immunized B78H1/H11 survived 12 weeks (Fig. 19B). These experiments indicated that anti-primary tumor activity of H11 might be associated with non-specific immunological reaction.

In the second set of experiments analyses of induction and effector phases of anti melanoma immune responses elicited by IL-11 and H11 gene modified tumor vaccines were carried out. The induction phase was studied by injecting control and GMTV into unprimed mice and analysis of immune cell infiltration (immunohistochemistry). Control B78H1 cells were infiltrated by single CD4+ and CD8+ T lymphocytes. GMTV-IL-11 cells were densely infiltrated by CD4+ and CD8+ cells, while none of them was detected in B78H1/H11 vaccinated mice. These results indicated that induction phase of anti-melanoma response elicited by IL-11 and H11 GMTV is mediated by different mechanisms. Moreover, B78H1/H11 cells were densely infiltrated by NK cells what confirmed the important role of these cells in the growth inhibition of the primary tumor. In the effector phase, in which mice pre-immunized with control GMTV were re-challenged with parental B78H1 cells, infiltrates of single CD4+ cells were observed. However, B78H1 tumors in mice immunized with IL-11-GMTV were densely infiltrated by CD4+ and CD8+, while immunization with B78H1/H11 caused heavy infiltration of NK cells. These data indicated that CD4+ and in the late phase CD8+ lymphocytes are the major effector cells stimulated by IL-11-GMTV, while H11-GMTV activates NK and in the late phase CD4+ cells.

Figure 20 and 21 summarize analyses of induction and effector phases of anti melanoma immune responses elicited by IL-11- and H11-GMTV.

### References

Altschul SF, Gish W, Miller W, Myers EW, Lipman DJ. Basic local alignment search tool. J Mol Biol. 1990 Oct 5;215(3):403-10.
Altschul SF, Madden TL, Schaffer AA, Zhang J, Zhang Z, Miller W, Lipman DJ. Gapped BLAST and PSI-BLAST: a new generation of protein database search programs. Nucleic Acids Res. 1997 Sep 1;25(17):3389-402.
Anguita J, Barthold SW, Samanta S, Ryan J, Fikrig E. Selective anti-inflammatory action of interleukin-11 in murine Lyme disease: arthritis decreases while carditis persists. J Infect Dis. 1999 Mar; 179(3): 734-7.
Barker JL, Montague ED, Peters LJ. Clinical experience with irradiation of inflammatory carcinoma of the breast with and without elective chemotherapy. Cancer. 1980 Feb 15;45(4):625-9.
Barton VA, Hudson KR, Heath JK. Identification of three distinct receptor binding sites of murine interleukin-11. J Biol Chem. 1999 Feb 26;274(9):5755-61.
Barton VA, Hall MA, Hudson KR, Heath JK. Interleukin-11 signals through the formation of a hexameric receptor complex. J Biol Chem. 2000 Nov 17;275(46):36197-203.
Baumann H, Schendel P. Interleukin-11 regulates the hepatic expression of the same plasma protein genes as interleukin-6. J Biol Chem. 1991 Oct 25; 266(30): 20424-7.
Baumann H, Wang Y, Morella KK, Lai CF, Dams H, Hilton DJ, Hawley RG, Mackiewicz A. Complex of the soluble IL-11 receptor and IL-11 acts as IL-6-type cytokine in hepatic and nonhepatic cells. J Immunol. 1996 Jul 1;157(1):284-90.
Bazan JF. Structural design and molecular evolution of a cytokine receptor superfamily. Proc Natl Acad Sci U S A. 1990 Se; 87(18):6934-8.
Bozza M, Bliss JL, Maylor R, Erickson J, Donnelly L, Bouchard P, Dorner AJ, Trepicchio WL. Interleukin-11 reduces T-cell-dependent experimental liver injury in mice. Hepatology. 1999 Dec; 30(6): 1441-7.
Bravo J, Heath JK. Receptor recognition by gp130 cytokines. EMBO J. 2000 Jun 1;19(11):2399-411.
Carlezon WA Jr, Nestler EJ, Neve RL. Herpes simplex virus-mediated gene transfer as a tool for neuropsychiatric research. Crit Rev Neurobiol. 2000;14(1):47-67.
Carter PJ, Samulski RJ. Adeno-associated viral vectors as gene delivery vehicles. Int J Mol Med. 2000 Jul; 6(1):17-27.
Chang M, Williams A, Ishizawa L, Knoppel A, van de Ven C, Cairo MS. Endogenous interleukin-11 (IL-11) expression is increased and prophylactic use of exogenous IL-11 enhances platelet recovery and improves survival during thrombocytopenia associated with experimental group B streptococcal sepsis in neonatal rats. : Blood Cells Mol Dis. 1996; 22(1): 57-67.
Chang LJ, Gay EE. The molecular genetics of lentiviral vectors--current and future perspectives. Curr Gene Ther. 2001 Sep; 1(3):237-51.
Cherel M, Sorel M, Lebeau B, Dubois S, Moreau JF, Bataille R, Minvielle S, Jacques Y. Molecular cloning of two isoforms of a receptor for the human hematopoietic cytokine interleukin-11. Blood. 1995 Oct 1;86(7):2534-40.
Cherel M, Sorel M, Apiou F, Lebeau B, Dubois S, Jacques Y, Minvielle S. The human interleukin-11 receptor alpha gene (IL11RA): genomic organization and chromosome mapping. Genomics. 1996 Feb 15;32(1):49-53.
Chomczynski P, Sacchi N. Single-step method of RNA isolation by acid guanidinium thiocyanate-phenol-chloroform extraction. Anal Biochem. 1987 Apr;162(1):156-9.
Curtis DJ, Hilton DJ, Roberts B, Murray L, Nicola N, Begley CG. Recombinant soluble interleukin-11 (IL-11) receptor alpha-chain can act as an IL-11 antagonist. Blood. 1997 Dec 1;90(11):4403-12.
Czupryn MJ, McCoy JM, Scoble HA. Structure-function relationships in human interleukin-11. Identification of regions involved in activity by chemical modification and site-directed mutagenesis. J Biol Chem. 1995 Jan 13;270(2):978-85.
Dams-Kozlowska H, Izycki D, Mackiewicz A. IL-11 is a potent anti-melanoma factor. Adv Exp Med Biol. 2001;495:373-7.
Davis S, Aldrich TH, Valenzuela DM, Wong VV, Furth ME, Squinto SP, Yancopoulos GD. The receptor for ciliary neurotrophic factor. Science. 1991 Jul 5;253(5015):59-63.
Deller MC, Hudson KR, Ikemizu S, Bravo J, Jones EY, Heath JK. Crystal structure and functional dissection of the cytostatic cytokine oncostatin M. Structure Fold Des. 2000 Aug 15;8(8):863-74.
Dimitriadis E, Salamonsen LA, Robb L. Expression of interleukin-11 during the human menstrual cycle: coincidence with stromal cell decidualization and relationship to leukaemia inhibitory factor and prolactin. : Mol Hum Reprod. 2000 Oct; 6(10): 907-14.
Du XX, Neben T, Goldman S, Williams DA. Effects of recombinant human interleukin-11 on hematopoietic reconstitution in transplant mice: acceleration of recovery of peripheral blood neutrophils and platelets. Blood. 1993 Jan 1; 81(1): 27-34.
Du X, Williams DA. Interleukin-11: review of molecular, cell biology, and clinical use. Blood. 1997 Jun 1;89(11):3897-908.
Dull T, Zufferey R, Kelly M, Mandel RJ, Nguyen M, Trono D, Naldini L. A third-generation lentivirus vector with a conditional packaging system. J Virol. 1998 Nov;72(11):8463-71.
Fastenberg NA, Martin RG, Buzdar AU, Hortobagyi GN, Montague ED, Blumenschein GR, Jessup JM. Management of inflammatory carcinoma of the breast. A combined modality approach. Am J Clin Oncol. 1985 Apr;8(2): 134-41.
Fischer M, Goldschmitt J, Peschel C, Brakenhoff JP, Kallen KJ, Wollmer A, Grotzinger J, Rose-John S. I. A bioactive designer cytokine for human hematopoietic progenitor cell expansion. Nat Biotechnol. 1997 Feb;15(2):142-5.
Girasole G, Passeri G, Jilka RL, Manolagas SC. Interleukin-11: a new cytokine critical for osteoclast development. J Clin Invest. 1994 Apr; 93(4): 1516-24.
Gordon MS, McCaskill-Stevens WJ, Battiato LA, Loewy J, Loesch D, Breeden E, Hoffman R, Beach KJ, Kuca B, Kaye J, Sledge GW Jr. A phase I trial of recombinant human interleukin-11 (neumega rhIL-11 growth factor) in women with breast cancer receiving chemotherapy. Blood. 1996 May 1; 87(9): 3615-24.
Grotzinger J, Kurapkat G, Wollmer A, Kalai M, Rose-John S. The family of the IL-6-type cytokines: specificity and promiscuity of the receptor complexes. Proteins. 1997 Jan;27(1):96-109.
Grotzinger J, Kernebeck T, Kallen KJ, Rose-John S. IL-6 type cytokine receptor complexes: hexamer, tetramer or both? Biol Chem. 1999 Jul-Aug;380(7-8):803-13.
Harmegnies D, Wang XM, Vandenbussche P, Leon A, Vusio P, Grotzinger J, Jacques Y, Goormaghtigh E, Devreese B, Content J. Characterization of a potent human interleukin-11 agonist. Biochem J. 2003 Oct 1;375(Pt 1):23-32.
Hawley RG, Hawley TS, Fong AZ, Quinto C, Collins M, Leonard JP, Goldman SJ. Thrombopoietic potential and serial repopulating ability of murine hematopoietic stem cells constitutively expressing interleukin 11. Proc Natl Acad Sci U S A. 1996 Sep 17; 93(19): 10297-302.
Heinrich PC, Behrmann I, Haan S, Hermanns HM, Muller-Newen G, Schaper F. Principles of interleukin (IL)-6-type cytokine signalling and its regulation. Biochem J. 2003 Aug 15;374(Pt 1):1-20.
Hilton DJ, Hilton AA, Raicevic A, Rakar S, Harrison-Smith M, Gough NM, Begley CG, Metcalf D, Nicola NA, Willson TA. Cloning of a murineeceptor alpha-chain; requirement for gp130 for high affinity binding and signal transduction. EMBO J. 1994 Oct 17;13(20):4765-75.
Isaacs C, Robert NJ, Bailey FA, Schuster MW, Overmoyer B, Graham M, Cai B, Beach KJ, Loewy JW, Kaye JA. Randomized placebo-controlled study of recombinant human interleukin-11 to prevent chemotherapy-induced thrombocytopenia in patients with breast cancer receiving dose-intensive cyclophosphamide and doxorubicin. J Clin Oncol. 1997 Nov; 15(11): 3368-77.
Karow J, Hudson KR, Hall MA, Vernallis AB, Taylor JA, Gossler A, Heath JK. Mediation of interleukin-11-dependent biological responses by a soluble form of the interleukin-11 receptor. Biochem J. 1996 Sep 1;318 (Pt 2):489-95.
Karpovich N, Chobotova K, Carver J, Heath JK, Barlow DH, Mardon HJ. Expression and function of interleukin-11 and its receptor alpha in the human endometrium. Mol Hum Reprod. 2003 Feb; 9(2): 75-80.
Karlin S, Altschul SF. Applications and statistics for multiple high-scoring segments in molecular sequences. Proc Natl Acad Sci U S A. 1993 Jun 15;90(12):5873-7.
Kobinger GP, Weiner DJ, Yu QC, Wilson JM. Filovirus-pseudotyped lentiviral vector can efficiently and stably transduce airway epithelia in vivo. Nat Biotechnol. 2001 Mar;19(3):225-30.
Lebeau B, Montero Julian FA, Wijdenes J, Muller-Newen G, Dahmen H, Cherel M, Heinrich PC, Brailly H, Hallet MM, Godard A, Minvielle S, Jacques Y. Reconstitution of two isoforms of the human interleukin-11 receptor and comparison of their functional properties. FEBS Lett. 1997 Apr 28;407(2):141-7.
Leng SX, Elias JA. Interleukin-11 inhibits macrophage interleukin-12 production. J Immunol. 1997 Sep 1; 159(5): 2161-8.
Lieschke GJ, Rao PK, Gately MK, Mulligan RC. Bioactive murine and human interleukin-12 fusion proteins which retain antitumor activity *in vivo.* Nat Biotechnol. 1997 Jan; 15(1): 35-40.
Lindemann D, Patriquin E, Feng S, Mulligan RC. Versatile retrovirus vector systems for regulated gene expression in vitro and in vivo. Mol Med. 1997 Jul;3(7):466-76.
Mackiewicz A, Schooltink H, Heinrich PC, Rose-John S. Complex of soluble human IL-6-receptor/IL-6 up-regulates expression of acute-phase proteins. J Immunol. 1992 Sep 15;149(6):2021-7.
Mackiewicz A, Wiznerowicz M, Roeb E, Karczewska A, Nowak J, Heinrich PC, Rose-John S. Soluble interleukin 6 receptor is biologically active *in vivo.* Cytokine. 1995a Feb;7(2):142-9.
Mackiewicz A, Wiznerowicz M, Roeb E, Nowak J, Pawlowski T, Baumann H, Heinrich PC, Rose-John S. Interleukin-6-type cytokines and their receptors for gene therapy of melanoma. Ann N Y Acad Sci. 1995b Jul 21;762:361-73; discussion 373-4.
Mackiewicz A, Gorny A, Laciak M, Malicki J, Murawa P, Nowak J, Wiznerowicz M, Hawley RG, Heinrich PC, Rose-John S. Gene therapy of human melanoma. Immunization of patients with autologous tumor cells admixed with allogeneic melanoma cells secreting interleukin 6 and soluble interleukin 6 receptor. Hum Gene Ther. 1995c Jun; 6(6): 805-11.
Maier R, Ganu V, Lotz M. Interleukin-11, an inducible cytokine in human articular chondrocytes and synoviocytes, stimulates the production of the tissue inhibitor of metalloproteinases. J Biol Chem. 1993 Oct 15; 268(29): 21527-32.
Marz P, Otten U, Rose-John S. Neural activities of IL-6-type cytokines often depend on soluble cytokine receptors. Eur J Neurosci. 1999 Sep;11 (9):2995-3004.
McDonald NQ, Panayotatos N, Hendrickson WA. Crystal structure of dimeric human ciliary neurotrophic factor determined by MAD phasing. EMBO J. 1995 Jun 15;14(12):2689-99.
Mehler MF, Rozental R, Dougherty M, Spray DC, Kessler JA. Cytokine regulation of neuronal differentiation of hippocampal progenitor cells. Nature. 1993 Mar 4; 362(6415): 62-5.
Nandurkar HH, Hilton DJ, Nathan P, Willson T, Nicola N, Begley CG. The human IL-11 receptor requires gp130 for signalling: demonstration by molecular cloning of the receptor. Oncogene. 1996 Feb 1;12(3):585-93.
Neddermann P, Graziani R, Ciliberto G, Paonessa G. Functional expression of soluble human interleukin-11 (IL-11) receptor alpha and stoichiometry of *in vitro* IL-11 receptor complexes with gp130. J Biol Chem. 1996 Nov 29;271(48):30986-91.
Ohsumi J, Miyadai K, Kawashima I, Sakakibara S, Yamaguchi J, Itoh Y. Regulation of lipoprotein lipase synthesis in 3T3-L1 adipocytes by interleukin-11/adipogenesis inhibitory factor. Biochem Mol Biol Int. 1994 Mar; 32(4): 705-12.
Ozbek S, Grotzinger J, Krebs B, Fischer M, Wollmer A, Jostock T, Mullberg J, Rose-John S. The membrane proximal cytokine receptor domain of the human interleukin-6 receptor is sufficient for ligand binding but not for gp130 association. J Biol Chem. 1998 Aug 14;273(33):21374-9.
Paul SR, Bennett F, Calvetti JA, Kelleher K, Wood CR, O'Hara RM Jr, Leary AC, Sibley B, Clark SC, Williams DA*, et al.* Molecular cloning of a cDNA encoding interleukin 11, a stromal cell-derived lymphopoietic and hematopoietic cytokine. Proc Natl Acad Sci U S A. 1990 Oct;87(19):7512-6.
Peterson RL, Wang L, Albert L, Keith JC Jr, Dorner AJ. Molecular effects of recombinant human interleukin-11 in the HLA-B27 rat model of inflammatory bowel disease. Lab Invest. 1998 Dec; 78(12): 1503-12.
Pflanz S, Tacken I, Grotzinger J, Jacques Y, Minvielle S, Dahmen H, Heinrich PC, Muller-Newen G. A fusion protein of interleukin-11 and soluble interleukin-11 receptor acts as a superagonist on cells expressing gp130. FEBS Lett. 1999 Apr 30;450(1-2):117-22.
Redlich CA, Gao X, Rockwell S, Kelley M, Elias JA. IL-11 enhances survival and decreases TNF production after radiation-induced thoracic injury. J Immunol. 1996 Aug 15; 157(4): 1705-10.
Robb L, Hilton DJ, Willson TA, Begley CG. Structural analysis of the gene encoding the murine interleukin-11 receptor alpha-chain and a related locus. J Biol Chem. 1996 Jun 7;271(23):13754-61.
Robb L, Li R, Hartley L, Nandurkar HH, Koentgen F, Begley CG. Infertility in female mice lacking the receptor for interleukin 11 is due to a defective uterine response to implantation. Nat Med. 1998 Mar; 4(3): 303-8.
Robinson RC, Grey LM, Staunton D, Vankelecom H, Vernallis AB, Moreau JF, Stuart DI, Heath JK, Jones EY. The crystal structure and biological function of leukemia inhibitory factor: implications for receptor binding. Cell. 1994 Jul 1;77(7):1101-16.
Rose-John S, Schooltink H, Lenz D, Hipp E, Dufhues G, Schmitz H, Schiel X, Hirano T, Kishimoto T, Heinrich PC. Studies on the structure and regulation of the human hepatic interleukin-6 receptor. Eur J Biochem. 1990 May 31;190(1):79-83.
Schleinkofer K, Dingley A, Tacken I, Federwisch M, Muller-Newen G, Heinrich PC, Vusio P, Jacques Y, Grotzinger J. Identification of the domain in the human interleukin-11 receptor that mediates ligand binding. J Mol Biol. 2001 Feb 16;306(2):263-74.
Schwertschlag US, Trepicchio WL, Dykstra KH, Keith JC, Turner KJ, Dorner AJ. Hematopoietic, immunomodulatory and epithelial effects of interleukin-11. Leukemia. 1999 Sep; 13(9): 1307-15.
Somers W, Stahl M, Seehra JS. 1.9 A crystal structure of interleukin 6: implications for a novel mode of receptor dimerization and signaling. EMBO J. 1997 Mar 3;16(5):989-97.
Sonis ST, Van Vugt AG, McDonald J, Dotoli E, Schwertschlag U, Szklut P, Keith J. Mitigating effects of interleukin 11 on consecutive courses of 5-fluorouracil-induced ulcerative mucositis in hamsters. Cytokine. 1997 Aug; 9(8): 605-12.
Springer ML, Chen AS, Kraft PE, Bednarski M, Blau HM. VEGF gene delivery to muscle: potential role for vasculogenesis in adults. Mol Cell. 1998 Nov;2(5):549-58.
Tacken I, Dahmen H, Boisteau O, Minvielle S, Jacques Y, Grotzinger J, Kuster A, Horsten U, Blanc C, Montero-Julian FA, Heinrich PC, Muller-Newen G. Definition of receptor binding sites on human interleukin-11 by molecular modeling-guided mutagenesis. Eur J Biochem. 1999 Oct;265(2):645-55.
Tepler I, Elias L, Smith JW 2nd, Hussein M, Rosen G, Chang AY, Moore JO, Gordon MS, Kuca B, Beach KJ, Loewy JW, Garnick MB, Kaye JA. A randomized placebo-controlled trial of recombinant human interleukin-11 in cancer patients with severe thrombocytopenia due to chemotherapy. Blood. 1996 May 1; 87(9): 3607-14.
Trepicchio WL, Bozza M, Pedneault G, Dorner AJ. Recombinant human IL-11 attenuates the inflammatory response through down-regulation of proinflammatory cytokine release and nitric oxide production. J Immunol. 1996 Oct 15; 157(8): 3627-34.
Trepicchio WL, Wang L, Bozza M, Dorner AJ. IL-11 regulates macrophage effector function through the inhibition of nuclear factor-kappaB. J Immunol. 1997 Dec 1; 159(11): 5661-70.
Trepicchio WL, Ozawa M, Walters IB, Kikuchi T, Gilleaudeau P, Bliss JL, Schwertschlag U, Dorner AJ, Krueger JG. Interleukin-11 therapy selectively downregulates type I cytokine proinflammatory pathways in psoriasis lesions. J Clin Invest. 1999 Dec; 104(11): 1527-37.
Underhill-Day N, McGovern LA, Karpovich N, Mardon HJ, Barton VA, Heath JK. Functional characterization of W147A: a high-affinity interleukin-11 antagonist. Endocrinology. 2003 Aug;144(8):3406-14.
Walmsley M, Butler DM, Marinova-Mutafchieva L, Feldmann M. An anti-inflammatory role for interleukin-11 in established murine collagen-induced arthritis. Immunology. 1998 Sep; 95(1): 31-7.
Wells JA. Binding in the growth hormone receptor complex. Proc Natl Acad Sci U S A. 1996 Jan 9;93(1):1-6.
Wiznerowicz M, Fong AZ, Mackiewicz A, Hawley RG. Double-copy bicistronic retroviral vector platform for gene therapy and tissue engineering: application to melanoma vaccine development. Gene Ther. 1997 Oct;4(10):1061-8.
Vollmer P, Oppmann B, Voltz N, Fischer M, Rose-John S. A role for the immunoglobulin-like domain of the human IL-6 receptor. Intracellular protein transport and shedding. Eur J Biochem. 1999 Jul;263(2):438-46.
de Vos AM, Ultsch M, Kossiakoff AA. Human growth hormone and extracellular domain of its receptor: crystal structure of the complex. Science. 1992 Jan 17;255(5042):306-12.
Yawata H, Yasukawa K, Natsuka S, Murakami M, Yamasaki K, Hibi M, Taga T, Kishimoto T. Structure-function analysis of human IL-6 receptor: dissociation of amino acid residues required for IL-6-binding and for IL-6 signal transduction through gp130. EMBO J. 1993 Apr; 12(4):1705-12.
Zufferey R, Nagy D, Mandel RJ, Naldini L, Trono D. Multiply attenuated lentiviral vector achieves efficient gene delivery in vivo. Nat Biotechnol. 1997 Sep;15(9):871-5. Related Articles, Links

## Claims

1. A polynucleotide selected from the group consisting of:
(a) polynucleotides encoding a fusion interleukin-11 receptor (IL-11 R) and IL-11 polypeptide (H11) comprising at least the soluble IL-11 R having the deduced amino acid sequence as shown in SEQ ID NO: 1 and a mature IL-11 having the deduced amino acid sequence as shown in SEQ ID NO: 2;
(b) polynucleotides comprising the coding sequence of IL-11 R, as shown in SEQ ID NO: 4 and the coding sequence of IL-11, as shown in SEQ ID NO: 5 encoding H11;
(c) polynucleotides encoding a fragment and/or derivative of a H11 encoded by a polynucleotide of any one of (a) to (b), wherein in said derivative one or more amino acid residues are conservatively substituted compared to said H11 and said fragment and/or derivative has *in vivo* anti-tumor activity;
(d) polynucleotides which are at least 50% identical to a polynucleotide as defined in any one of (a) to (c) and which code for a H11 having *in vivo* anti-tumor activity; and
(e) polynucleotides the complementary strand of which hybridizes, preferably under stringent conditions to a polynucleotide as defined in any one of (a) to (d) and which code for a H11 having *in vivo* anti-tumor activity;
or the complementary strand of such a polynucleotide.

2. The polynucleotide of claim 1, wherein the polynucleotide encoding the soluble IL-11 R is positioned 5' terminal with respect to the polynucleotide encoding mature IL-11.

3. The polynucleotide of claim 1 or 2, wherein the nucleotide sequence intervening the polynucleotide sequences encoding the soluble IL-11 R and the mature IL-11 encodes a non-immunogenic peptide.

4. The polynucleotide of claim 1 or 2, wherein the polynucleotides encoding soluble IL-11 R and mature IL-11 are directly linked.

5. The polynucleotide of claim 4, wherein the polynucleotide is selected from the group consisting of:
(a) polynucleotides encoding H11 having the deduced amino acid sequence as shown in SEQ ID NO: 3;
(b) polynucleotides comprising the coding sequence of H11, as shown in SEQ ID NO: 6;
(c) polynucleotides encoding a fragment and/or derivative of a H11 encoded by a polynucleotide of any one of (a) to (b), wherein in said derivative one or more amino acid residues are conservatively substituted compared to said H11 and said fragment and/or derivative has *in vivo* anti-tumor activity;
(d) polynucleotides which are at least 50% identical to a polynucleotide as defined in any one of (a) to (c) and which code for a H11 having *in vivo* anti-tumor activity; and
(e) polynucleotides the complementary strand of which hybridizes, preferably under stringent conditions to a polynucleotide as defined in any one of (a) to (d) and which code for a H11 having *in vivo* anti-tumor activity;
or the complementary strand of such a polynucleotide.

6. The polynucleotide of one of claims 1 to 5, which is DNA, cDNA, genomic DNA, synthetic DNA or RNA.

7. A vector containing the polynucleotide of one of claims 1 to 6.

8. The vector of claim 7 in which the polynucleotide is operatively linked to expression control sequences allowing expression in prokaryotic and/or eukaryotic host cells.

9. The vector of claim 7 or 8, wherein the expression control sequences are selected from the group consisting of CMV, SV40, polyhedrin promoter, retroviral LTRs, phosphoglycerate kinase (PGK), elongation factor 1-α (EF 1-α) and phosphoenolpyruvate carboxykinase (PEPCK).

10. The vector of one of claims 7 to 9, wherein the vector is selected from the group consisting of a plasmid; phagemid; phage; cosmid; artificial mammalian chromosome; artificial yeast chromosome; knock-out or knock-in construct; virus, in particular adenovirus, vaccinia virus, attenuated vaccinia virus, canary pox virus, lentivirus, herpes virus, in particular Herpes simplex virus, baculovirus, retrovirus, adeno-associated-virus (AAV), rhinovirus, human immune deficiency virus (HIV), filovirus and engineered versions thereof; virosomes, virus-like particles and liposomes.

11. A host cell genetically engineered with the polynucleotide of one of claims 1 to 6 or the vector of one of claims 7 to 10.

12. The host cell of claim 11, wherein the host cell is selected from the group consisting of insect cells, in particular *Spodoptera frugiperda* and *Trichoplasia ni,* mammalian cells, in particular stem cells, hemopoietic cells, hepatocytes, adipocytes, neurons, osteoclosts, uterine endometrium cells, dermatocytes, myocardial cells, mucosal cells or tumor cells; bacterial cells, in particular of the *Escherichia* or *Bacillus* species and yeast cells, in particular of *Pischia* or *Saccharomyces* species.

13. The host cell of claims 11 or 12, wherein the host cell is selected from the group consisting of renal cancer cells, pancreatic cancer cells, leucocytes, melanoma cells, packaging cells, in particular amphotropic or ecotropic packaging cells.

14. The host cell of any of claims 11 to 13, which is genetically engineered to express at least one further polynucleotide.

15. The host cell of claim 14, wherein the further polynucleotide encodes a cytokine, in particular GM-CSF, IL-6, IL-11, IL-15, anti-TGF, EPO, interferons, LIF, OSM, CNTF, CT-1 and sIL-6 R/IL-6 fusion proteins, in particular Hyper IL-6.

16. A process for producing cells capable of expressing H11 comprising genetically engineering cells *in vitro* with the vector of one of claims 7 to 10, wherein said H11 is encoded by the polynucleotide of one of claims 1 to 6.

17. A process for producing a H11 polypeptide encoded by the polynucleotide of one of claims 1 to 6 comprising: culturing the host cell of one of claims 11 to 15 and recovering the H11 polypeptide encoded by said polynucleotide.

18. A H11 polypeptide having the amino acid sequence encoded by the polynucleotide of one of claims 1 to 6 or obtainable by the process of claim 17.

19. An antibody specific to the polypeptide encoded by the polynucleotide of one of claims 1 to 6 or obtainable by the process of claim 17, which is essentially non-specific to soluble IL-11R and IL-11.

20. A pharmaceutical composition comprising the host cell of one of claims 11 to 15 or obtainable according to claim 16 or the H11 of claim 18 or obtainable according to claim 17, further comprising excipients, stabilizers, protectants, buffers and/or additives.

21. Use of the host cell of one of claims 11 or 15 or obtainable according to claim 16 or the H11 of claim 18 for the manufacture of a medicament for the treatment of a disease selected from the group consisting of a proliferative disease, a cytopathy, radiation damage, an IL-11 dependent inflammatory disorder, IL-11 dependent degenerative disorder and IL-11 dependent or mediated soft tissue disorder.

22. The use of claim 21, wherein the proliferative disease is selected from the group consisting of cancer of the gastrointestinal or colorectal tract, liver, pancreas, kidney, bladder, prostate, endometrium, ovary, testes, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, glioma, astrocytoma, osteosarcoma, soft tissue sarcoma, hemangioma, endocrinological tumor, hematologic neoplasia including leukemia, lymphoma, and other myeloproliferative and lymphoproliferative diseases, carcinoma in situ, hyperplastic lesion, adenoma, fibroma, histiocytosis, chronic inflammatory proliferative disease, vascular proliferative disease and virus-induced proliferative disease; in particular melanoma, pancreatic and renal cancer

23. The use of claim 21, wherein the cytopathy is selected form the group consisting of thrombocytopenia, hematocytopenia, and pancytopenia.

24. The use of claim 21, wherein the IL-11 dependent inflammatory disorder is selected from the group consisting of liver failure; hepatitis; hepatopathy; sepsis; chemotherapy or radiation-induced tissue damage, in particular lung damage; inflammatory diseases, in particular inflammatory bowel disease, rheumatoid arthritis, inflammatory liver disease; mucositis; allergies; endometriosis; vasculitis; vascular disease associated with endothelial inflammation, in particular ischaemic heart diseases or peripheral vascular disease; and psoriasis.

25. The use of claim 21, wherein the IL-11 dependent degenerative disease is selected from the group consisting of degenerative CNS disease, PNS disease and osteoarthritis.

26. The use of claim 21, wherein the IL-11 dependent or mediated soft tissue disorder is selected from the group consisting of obesity and idiopathic female infertility.

27. The use of any one of claims 21 to 26, wherein at least one further cytokine is administered prior, simultaneously or subsequently to the administration of H11 polypeptide or a host cell expressing H11 polypeptide.

28. Use of IL-11 of claim 18 or obtainable according to the process of claim 17 for the manufacture of a medicament for adjuvant therapy during stem cell therapy.

29. Use of IL-11 of claim 18 or obtainable according to the process of claim 17 for the *in vitro* differentiation of cells, in particular stem cells or precursor cells.


SEQUENCE LISTING

<110>  AGIRx Limited

<120>  Chimeric soluble hyper IL-11 and use thereof

<130>  FB13498

<160>  12

<170>  PatentIn version 3.2

<210>  1
<211>  365
<212>  PRT
<213>  Artificial

<220>
<223>  Encoding human mutant soluble IL-11 R fragement

<400>  1

Met Gly Ser Ser Cys Ser Gly Leu Ser Arg Val Leu Val Ala Val Ala
1               5                   10                  15

Thr Ala Leu Val Ser Ala Ser Ser Pro Cys Pro Gln Ala Trp Gly Pro
            20                  25                  30

Pro Gly Val Gln Tyr Gly Gln Pro Gly Arg Ser Val Lys Leu Cys Cys
        35                  40                  45

Pro Gly Val Thr Ala Gly Asp Pro Val Ser Trp Phe Arg Asp Gly Glu
    50                  55                  60

Pro Lys Leu Leu Gln Gly Pro Asp Ser Gly Leu Gly His Glu Leu Val
65                  70                  75                  80

Leu Ala Gln Ala Asp Ser Thr Asp Glu Gly Thr Tyr Ile Cys Gln Thr
                85                  90                  95

Leu Asp Gly Ala Leu Gly Gly Thr Val Thr Leu Gln Leu Gly Tyr Pro
            100                 105                 110

Pro Ala Arg Pro Val Val Ser Cys Gln Ala Ala Asp Tyr Glu Asn Phe
        115                 120                 125

Ser Cys Thr Trp Ser Pro Ser Gln Ile Ser Gly Leu Pro Thr Arg Tyr
    130                 135                 140

Leu Thr Ser Tyr Arg Lys Lys Thr Val Leu Gly Ala Asp Ser Gln Arg
145                 150                 155                 160

Arg Ser Pro Ser Thr Gly Pro Trp Pro Cys Pro Gln Asp Pro Leu Gly

                165                 170                 175

Ala Ala Arg Cys Val Val His Gly Ala Glu Phe Trp Ser Gln Tyr Arg
            180                 185                 190

Ile Asn Val Thr Glu Val Asn Pro Leu Gly Ala Ser Thr Arg Leu Leu
        195                 200                 205

Asp Val Ser Leu Gln Ser Ile Leu Arg Pro Asp Pro Pro Gln Gly Leu
    210                 215                 220

Arg Val Glu Ser Val Pro Gly Tyr Pro Arg Arg Leu Arg Ala Ser Trp
225                 230                 235                 240

Thr Tyr Pro Ala Ser Trp Pro Cys Gln Pro His Phe Leu Leu Lys Phe
                245                 250                 255

Arg Leu Gln Tyr Arg Pro Ala Gln His Pro Ala Trp Ser Thr Val Glu
            260                 265                 270

Pro Ala Gly Leu Glu Glu Val Ile Thr Asp Ala Val Ala Gly Leu Pro
        275                 280                 285

His Ala Val Arg Val Ser Ala Arg Asp Phe Leu Asp Ala Gly Thr Trp
    290                 295                 300

Ser Thr Trp Ser Pro Glu Ala Trp Gly Thr Pro Ser Thr Gly Thr Ile
305                 310                 315                 320

Pro Lys Glu Ile Pro Ala Trp Gly Gln Leu His Thr Gln Pro Glu Val
                325                 330                 335

Glu Pro Gln Val Asp Ser Pro Ala Pro Pro Arg Pro Ser Leu Gln Pro
            340                 345                 350

His Pro Arg Leu Leu Asp His Arg Asp Ser Val Glu Gln
        355                 360                 365

<210> 2
<211> 181
<212> PRT
<213> Artificial

<220>
<223> Fragement of human IL-11

<400> 2

Ala Val Ala Pro Gly Pro Pro Pro Gly Pro Pro Arg Val Ser Pro Asp
1               5                   10                  15

Pro Arg Ala Glu Leu Asp Ser Thr Val Leu Leu Thr Arg Ser Leu Leu
            20                  25                  30

Ala Asp Thr Arg Gln Leu Ala Ala Gln Leu Arg Asp Lys Phe Pro Ala
        35                  40                  45

Asp Gly Asp His Asn Leu Asp Ser Leu Pro Thr Leu Ala Met Ser Ala
    50                  55                  60

Gly Ala Leu Gly Ala Leu Gln Leu Pro Gly Val Leu Thr Arg Leu Arg
65                  70                  75                  80

Ala Asp Leu Leu Ser Tyr Leu Arg His Val Gln Trp Leu Arg Arg Ala
                85                  90                  95

Gly Gly Ser Ser Leu Lys Thr Leu Glu Pro Glu Leu Gly Thr Leu Gln
            100                 105                 110

Ala Arg Leu Asp Arg Leu Leu Arg Arg Leu Gln Leu Leu Met Ser Arg
        115                 120                 125

Leu Ala Leu Pro Gln Pro Pro Pro Asp Pro Pro Ala Pro Pro Leu Ala
    130                 135                 140

Pro Pro Ser Ser Ala Trp Gly Gly Ile Arg Ala Ala His Ala Ile Leu
145                 150                 155                 160

Gly Gly Leu His Leu Thr Leu Asp Trp Ala Val Arg Gly Leu Leu Leu
                165                 170                 175

Leu Lys Thr Arg Leu
            180

<210>  3
<211>  546
<212>  PRT
<213>  Artificial

<220>
<223>  Encoding fusion protein of human soluble mutant IL-11 R fragment
       and IL-11 fragment

<400>  3

<210>  4
<211>  1095
<212>  DNA
<213>  Artificial

<220>
<223>  Encoding human mutant soluble IL-11 R fragement

<400>  4
atgggcagca gctgctcagg gctgagcagg gtcctggtgg ccgtggctac agccctggtg      60

tctgcctcct ccccctgccc ccaggcctgg ggccccccag gggtccagta tgggcagcca     120

gggaggtccg tgaagctgtg ttgtcctgga gtgactgccg gggacccagt gtcctggttt     180

cgggatgggg agccaaagct gctccaggga cctgactctg ggctagggca tgaactggtc     240

ctggcccagg cagacagcac tgatgagggc acctacatct gccagaccct ggatggtgca     300

cttgggggca cagtgaccct gcagctgggc taccctccag cccgccctgt tgtctcctgc     360

caagcagccg actatgagaa cttctcttgc acttggagtc ccagccagat cagcggttta     420

cccacccgct acctcacctc ctacaggaag aagacagtcc taggagctga tagccagagg     480

aggagtccat ccacagggcc ctggccatgc ccacaggatc ccctaggggc tgcccgctgt     540

gttgtccacg gggctgagtt ctggagccag taccggatta atgtgactga ggtgaaccca     600

ctgggtgcca gcacacgcct gctggatgtg agcttgcaga gcatcttgcg ccctgaccca     660

ccccagggcc tgcgggtaga gtcagtacca ggttaccccc gacgcctgcg agccagctgg     720

acataccctg cctcctggcc gtgccagccc cacttcctgc tcaagttccg tttgcagtac     780

cgtccggcgc agcatccagc ctggtccacg gtggagccag ctggactgga ggaggtgatc     840

acagatgctg tggctgggct gccccatgct gtacgagtca gtgcccggga ctttctagat     900

gctggcacct ggagcacctg gagcccggag gcctggggaa ctccgagcac tgggaccata     960

ccaaaggaga taccagcatg gggccagcta cacacgcagc cagaggtgga gcctcaggtg    1020

gacagccctg ctcctccaag gccctccctc caaccacacc ctcggctact tgatcacagg    1080

gactctgtgg agcag                                                    1095

<210>  5
<211>  546
<212>  DNA
<213>  Artificial

<220>
<223>  Encoding human IL-11 fragment

<400>  5
gctgtcgccc ctgggccacc acctggcccc cctcgagttt ccccagaccc tcgggccgag      60

ctggacagca ccgtgctcct gacccgctct ctcctggcgg acacgcggca gctggctgca     120

cagctgaggg acaaattccc agctgacggg gaccacaacc tggattccct gcccaccctg     180

gccatgagtg cgggggcact gggagctcta cagctcccag gtgtgctgac aaggctgcga     240

gcggacctac tgtcctacct gcggcacgtg cagtggctgc gccgggcagg tggctcttcc     300

ctgaagaccc tggagcccga gctgggcacc ctgcaggccc gactggaccg gctgctgcgc     360

cggctgcagc tcctgatgtc ccgcctggcc ctgccccagc cacccccgga cccgccggcg     420

cccccgctgg cgcccccctc ctcagcctgg gggggcatca gggccgccca cgccatcctg     480

ggggggctgc acctgacact tgactgggcc gtgaggggac tgctgctgct gaagactcgg     540

ctgtga                                                                546


<210>  6
<211>  1641
<212>  DNA
<213>  Artificial

<220>
<223>  Encoding fusion protein of human soluble mutant IL-11 R fragment
       and IL-11 fragment

<400>  6
atgggcagca gctgctcagg gctgagcagg gtcctggtgg ccgtggctac agccctggtg      60

tctgcctcct ccccctgccc ccaggcctgg ggccccccag gggtccagta tgggcagcca     120

gggaggtccg tgaagctgtg ttgtcctgga gtgactgccg gggacccagt gtcctggttt     180

cgggatgggg agccaaagct gctccaggga cctgactctg ggctagggca tgaactggtc     240

ctggcccagg cagacagcac tgatgagggc acctacatct gccagaccct ggatggtgca     300

cttgggggca cagtgaccct gcagctgggc taccctccag cccgccctgt tgtctcctgc     360

caagcagccg actatgagaa cttctcttgc acttggagtc ccagccagat cagcggttta     420

cccacccgct acctcacctc ctacaggaag aagacagtcc taggagctga tagccagagg     480

aggagtccat ccacagggcc ctggccatgc ccacaggatc ccctaggggc tgcccgctgt     540

gttgtccacg gggctgagtt ctggagccag taccggatta atgtgactga ggtgaaccca     600

ctgggtgcca gcacacgcct gctggatgtg agcttgcaga gcatcttgcg ccctgaccca     660

ccccagggcc tgcgggtaga gtcagtacca ggttaccccc gacgcctgcg agccagctgg     720

acataccctg cctcctggcc gtgccagccc cacttcctgc tcaagttccg tttgcagtac     780

cgtccggcgc agcatccagc ctggtccacg gtggagccag ctggactgga ggaggtgatc     840

acagatgctg tggctgggct gccccatgct gtacgagtca gtgcccggga ctttctagat     900

gctggcacct ggagcacctg gagcccggag gcctggggaa ctccgagcac tgggaccata 960

ccaaaggaga taccagcatg gggccagcta cacacgcagc cagaggtgga gcctcaggtg 1020

gacagccctg ctcctccaag gccctccctc caaccacacc ctcggctact tgatcacagg 1080

gactctgtgg agcaggctgt cgcccctggg ccaccacctg gcccccctcg agtttcccca 1140

gaccctcggg ccgagctgga cagcaccgtg ctcctgaccc gctctctcct ggcggacacg 1200

cggcagctgg ctgcacagct gagggacaaa ttcccagctg acggggacca caacctggat 1260

tccctgccca ccctggccat gagtgcgggg gcactgggag ctctacagct cccaggtgtg 1320

ctgacaaggc tgcgagcgga cctactgtcc tacctgcggc acgtgcagtg gctgcgccgg 1380

gcaggtggct cttccctgaa gaccctggag cccgagctgg gcaccctgca ggcccgactg 1440

gaccggctgc tgcgccggct gcagctcctg atgtcccgcc tggccctgcc ccagccaccc 1500

ccggacccgc cggcgccccc gctggcgccc ccctcctcag cctggggggg catcagggcc 1560

gcccacgcca tcctgggggg gctgcacctg acacttgact gggccgtgag gggactgctg 1620

ctgctgaaga ctcggctgtg a 1641

<210> 7
<211> 18
<212> DNA
<213> Artificial

<220>
<223> PCR-Primer for amplification of IL-11 fragment

<400> 7
gctgctgccc ctgggcca 18

<210> 8
<211> 45
<212> DNA
<213> Artificial

<220>
<223> PCR-Primer for amplification of IL-11 fragment

<400> 8
tccgcggccg ctatggccga cgtcgactca cagccgagtc ttcag 45

<210> 9
<211> 40
<212> DNA
<213> Artificial

<220>
<223> PCR-Primer for amplification of IL-11 R fragment

<400> 9
acgcgtcgac gccaccatgg gcagcagctg ctcagggctg 40

<210>  10
<211>  57
<212>  DNA
<213>  Artificial

<220>
<223>  PCR-Primer for amplification of IL-11 R fragment

<400>  10
aactcgaggg gggccaggtg gtggcccagg ggcgacagcc tgctccacag agtccct          57


<210>  11
<211>  20
<212>  DNA
<213>  Homo sapiens

<400>  11
acccacccgc tacctcacct                                                   20


<210>  12
<211>  17
<212>  DNA
<213>  Homo sapiens

<400>  12
gtcaggagca cggtgct                                                      17